# EUROPEAN PATENT SPECIFICATION

(11) **EP 3 007 704 B1**
(45) Date of publication and mention of the grant of the patent: **06.01.2021**
(21) Application number: 14810926.7
(22) Date of filing: 13.06.2014
(51) Int. Cl.: A61K 31/7088, A61K 38/31, A61P 31/00, A61P 5/02, A61K 45/06, A61K 31/7115, A61K 31/712, A61K 31/7125, A61K 31/713, C07K 14/655, C07K 14/72, C12N 15/113

(54) **COMBINATION THERAPY FOR ACROMEGALY**
KOMBINATIONSTHERAPIE DER AKROMEGALIE
POLYTHÉRAPIE POUR L'ACROMÉGALIE

(30) Priority: 13.06.2013 US 201361834856 P
(43) Date of publication of application: 20.04.2016
(73) Proprietor: Antisense Therapeutics Ltd, Toorak VIC 3142 (AU)
(72) Inventor: TACHAS, George, Kew Victoria 3101 (AU)
(74) Representative: Høiberg P/S
(86) International application number: PCT/AU2014/000613
(87) International publication number: WO 2014/197938

(56) References cited:
- WO-A1-2013/113074
- WO-A2-2004/078922
- WO-A2-2007/141306
- WILKINSON-BERKA, J.L. ET AL.: 'An antisense oligonucleotide targeting the growth hormone receptor inhibits neovascularization in a mouse model of retinopathy' MOLECULAR VISION vol. 13, 29 August 2007, pages 1529 - 1538, XP055302167
- BOLLERSLEV, J. ET AL.: 'NEW DIRECTIONS IN PHARMACOLOGICAL TREATMENT OF ACROMEGALY' EXPERT OPINION ON INVESTIGATIONAL DRUGS vol. 18, no. 1, January 2009, pages 13 - 22, XP055303536
- NEGGERS, S. J. C. ET AL.: 'Somatostatin analogue and pegvisomant combination therapy for acromegaly' NATURE REVIEWS: ENDOCRINOLOGY vol. 5, October 2009, pages 546 - 552, XP055303632

## Description

### FIELD

The present disclosure relates to a method for treatment or prevention of diseases caused by and/or associated with an increased level of insulin-like growth factor I (IGF-I) that relies on administration of a Somatostatin analog having Somatostatin receptor agonistic activity in combination with an antisense oligonucleotide targeted to the growth hormone receptor (GHR).

### BACKGROUND

Growth hormone (GH), released by the pituitary, is a member of a cascade of hormones that regulate growth of the body and its organs. Secretion of GH into the bloodstream is followed by binding to growth hormone receptor (GHR) on many cell and organ types. Growth hormone signaling is mediated by this interaction. Growth hormone signaling causes the production of another hormone, insulin-like growth factor I (IGF-I), which is produced in the liver, adipose tissue, kidney and other organs and secreted into the bloodstream. About 75% of serum IGF-I is produced in the liver in response to GH stimulation. Many disorders are caused by and/or associated with increased GH levels and/or increased IGF-I levels in plasma and/or tissues including acromegaly, gigantism, retinopathy, macular degeneration, nephropathy, diabetes and cancers. The role of GH and IGF-I in these and other disorders is well recognized. The role of IGF-I in mediating many GH effects is well recognized and this interrelationship is referred to as the GH/IGF-I axis. In a normal feedback loop, IGF-I also causes the production of GH by the pituitary to be reduced. There is a need for treatments that reduce IGF-I levels in a subject, for example, more effectively, safely, conveniently and/or at reduced cost.

Somatostatin analogs having somatostatin receptor agonistic activity are approved in the treatment of acromegaly to reduce GH and serum IGF-I levels in a patient. For a review of current practices for the treatment of acromegaly see Guistina et al., 2011. Briefly, in acromegaly, surgery is first used in treatment to debulk the tumor and reduce the pituitary tumor's GH secretion and reduce production of IGF-I in the serum. Medicinal treatments are also used to reduce serum IGF-I and in some cases also reduce GH release. All treatments use a medicinal monotherapy or a combination therapy directed to different biological protein targets. First line medicinal treatment is with a Somatostatin agonist, and this treatment is started initially at low doses and escalated to doses that reduce GH and normalize the patients serum IGF-I. When a Somatostatin agonist treatment fails, a dopamine agonist can be used in combination with a Somatostatin agonist, or Somavert is used in combination with a Somatostatin agonist or Somavert is used as a monotherapy. In patients where first line therapy using SST has failed or in patients where Somavert monotherapy or combination with Somatostatin has failed, clinicians are seeking new more effective treatments.
WO 2004/078922 discloses oligonucleotides to GHR that inhibit GHR and IGF-I expression and their use to treat IGF-I related diseases. The reference discloses a large number of oligonucleotides and other drugs (including the Somatostatin agonist, Octreotide) that can be used in combination with the oligonucleotides of the invention for treating a number of disorders. The data shown therein and relating to Octreotide do not relate to a combination of an oligonucleotide targeted to GHR and Octreotide.

### SUMMARY

The present inventors have now made the surprising finding that a Somatostatin analog having Somatostatin receptor agonistic activity and an antisense oligonucleotide targeted to GHR act to produce additional activity to reduce insulin-like growth factor I (IGF-I) compared to Somatostatin receptor agonistic activity and/or act synergistically to reduce insulin-like growth factor I (IGF-I) levels in a subject. In other words, the combined administration of the Somatostatin agonist and the oligonucleotide to GHR exhibits enhanced therapeutic activity compared to the Somatostatin agonist. This is surprising, particularly considering that there are no expected synergies with using antisense drugs targeted to the GHr, and a Somatostatin agonist.

Accordingly, the present disclosure provides a method for treatment or prevention of a disease caused by and/or associated with IGF-I, the method comprising administering to a subject in need thereof, a Somatostatin agonist in combination with an oligonucleotide 8 to 80 nucleobases in length targeted to a nucleic acid encoding GHR so as to inhibit expression of the GHR, thereby reducing the level of IGF-I in the subject. In one embodiment of the present disclosure, the level of serum/plasma IGF-I is reduced. In one embodiment of the present disclosure, the disease is caused by and/or associated with an increased level of IGF-I.

In one embodiment of the present disclosure, the method further comprises identifying a subject in need of a reduction in said subject's GHR and/or IGF-I levels, for example serum/plasma IGF-I levels.
In one embodiment of the present disclosure, the disease is acromegaly, gigantism, diabetic retinopathy, diabetic nephropathy, or an IGF-I positive and/or IGF-I and/or GH responsive cancer such as prostate, myeloma, lung, breast, or colon cancer.

In one embodiment of the present disclosure, the Somatostatin agonist has a higher binding affinity for human Somatostatin sub-type receptor 1 than the other human Somatostatin sub-type receptors, for human Somatostatin sub-type receptor 2 than the other human Somatostatin sub-type receptors, for human Somatostatin sub-type receptor 3 than the other human Somatostatin sub-type receptors, for human Somatostatin sub-type receptor 4 than the other human Somatostatin sub-type receptors, or for human Somatostatin sub-type receptor 5 than the other human Somatostatin sub-type receptors; or wherein the Somatostatin agonist has a higher binding affinity for two or more of human Somatostatin receptor sub-types, for example, 1, 2, 3, 4 and/or 5.
In one embodiment of the present disclosure, the Somatostatin agonist is for example Octreotide, Lanreotide, or Pasireotide in short, medium or, longacting forms or Octreolin. In a further embodiment of the present disclosure, the Somatostatin agonist is a long acting release (LAR) form, for example, Octreotide-LAR (SandostatinTM-LAR), Lanreotide-LAR (Lanreotide Autogel), or Pasireotide-LAR, or is Lanreotide microparticles.

In one embodiment of the present disclosure, the Somatostatin agonist is: 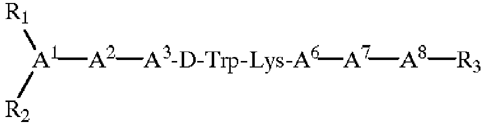 or a pharmaceutically acceptable salt thereof, wherein
A¹ is a D- or L-isomer of Ala, Leu, Ile, Val, Nle, Thr, Ser, β-Nal, β-Pal, Trp, Phe, 2,4-dichloro-Phe, pentafluoro-Phe, p-X-Phe, or o-X-Phe;
A² is Ala, Leu, Ile, Val, Nle, Phe, β-Nal, pyridyl-Ala, Trp, 2,4-dichloro-Phe, pentafluoro-Phe, o-X-Phe, or p-X-Phe;
A³ is pyridyl-Ala, Trp, Phe, β-Nal, 2,4-dichloro-Phe, pentafluoro-Phe, o-X-Phe, or p-X-Phe;
A⁶ is Val, Ala, Leu, Ile, Nle, Thr, Abu, or Ser;
A⁷ is Ala, Leu, Ile, Val, Nle, Phe, β-Nal, pyridyl-Ala, Trp, 2,4-dichloro-Phe, pentafluoro-Phe, o-X-Phe, or p-X-Phe;
A⁸ is a D- or L-isomer of Ala, Leu, Ile, Val, Nle, Thr, Ser, Phe, β-Nal, pyridyl-Ala, Trp, 2,4-dichloro-Phe, pentafluoro-Phe, p-X-Phe, or o-X-Phe;
wherein X for each occurrence is independently selected from the group consisting of CH₃, Cl, Br, F, OH, OCH₃ and NO₂;
each R₁ and R₂, independently, is H, lower acyl or lower alkyl; and R₃ is OH or NH2; provided that at least one of A¹ and A⁸ and one of A² and A⁷ must be an aromatic amino acid; and further provided that A¹, A², A⁷ and A⁸ cannot all be aromatic amino acids.

In another embodiment of the present disclosure, the Somatostatin agonist is:
H-D-Phe-p-chloro-Phe-Tyr-D-Trp-Lys-Thr-Phe-Thr-NH₂;
H-D-Phe-p-NO₂-Phe-Tyr-D-Trp-Lys-Val-Phe-Thr-NH₂;
H-D-Nal-p-chloro-Phe-Tyr-D-Trp-Lys-Val-Phe-Thr-NH₂;
H-D-Phe-Phe-Phe-D-Trp-Lys-Thr-Phe-Thr-NH₂;
H-D-Phe-Phe-Tyr-D-Trp-Lys-Val-Phe-Thr-NH₂;
H-D-Phe-p-chloro-Phe-Tyr-D-Trp-Lys-Val-Phe-Thr-NH₂; or
H-D-Phe-Ala-Tyr-D-Trp-Lys-Val-Ala-β-D-Nal-NH₂; or
a pharmaceutically acceptable salt thereof.

In another embodiment of the present disclosure, the Somatostatin agonist is:
D-Phe-Cys-Phe-D-Trp-Lys-Thr-Cys-β-Nal-NH₂;
D-Phe-Cys-Tyr-D-Trp-Lys-Thr-Cys-β-Nal-NH₂;
D-β-Nal-Cys-Phe-D-Trp-Lys-Thr-Cys-Thr-NH₂;
D-Phe-Cys-Tyr-D-Trp-Lys-Thr-Pen-Thr-NH₂;
D-Phe-Cys-Phe-D-Trp-Lys-Thr-Pen-Thr-NH₂;
D-Phe-Cys-Tyr-D-Trp-Lys-Thr-Pen-Thr-OH;
D-Phe-Cys-Phe-D-Trp-Lys-Thr-Pen-Thr-OH;
Gly-Pen-Phe-D-Trp-Lys-Thr-Cys-Thr-OH;
Phe-Pen-Tyr-D-Trp-Lys-Thr-Cys-Thr-OH;
Phe-Pen-Phe-D-Trp-Lys-Thr-Pen-Thr-OH;
H-D-Phe-Cys-Phe-D-Trp-Lys-Thr-Cys-Thr-ol;
H-D-Phe-Cys-Phe-D-Trp-Lys-Thr-Cys-Thr-NH₂;
H-D-Trp-Cys-Tyr-D-Trp-Lys-Val-Cys-Thr-NH₂;
H-D-Trp-Cys-Phe-D-Trp-Lys-Thr-Cys-Thr-NH₂;
H-D-Phe-Cys-Tyr-D-Trp-Lys-Val-Cys-Thr-NH₂;
H-D-Phe-Cys-Tyr-D-Trp-Lys-Val-Cys-Trp-NH₂;
H-D-Phe-Cys-Tyr-D-Trp-Lys-Val-Cys-Thr-NH₂;
Ac-D-Phe-Lys*-Tyr-D-Trp-Lys-Val-Asp-Thr-NH₂, wherein an amide bridge is between Lys* and Asp;
Ac-hArg(Et)₂-Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NH₂;
Ac-D-hArg(Bu)-Gly-Cys-Phe-D-Trp-Lys-Thr-Cys-Thr-NH₂;
Ac-D-hArg(Et)₂-Cys-Phe-D-Trp-Lys-Thr-Cys-Thr-H₂;
Ac-L-hArg(Et)₂-Cys-Phe-D-Trp-Lys-Thr-Cys-Thr-NH₂;
Ac-D-hArg(CH₂CF₃)₂-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Phe-NH₂;
Ac-D-hArg(CH₂CF₃)₂-Gly-Cys-Phe-D-Trp-Lys-Thr-Cys-Thr-NHEt;
Ac-L-hArg(CH₂CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys(Me)-Thr-Cys-Thr-NH₂;
Ac-D-hArg(CH₂CF₃)₂-Gly-Cys-Phe-D-Trp-Lys(Me)-Thr-Cys-Thr-NHEt;
Ac-hArg(CH₃, hexyl)-Gly-Cys-Phe-D-Trp-Lys-Thr-Cys-Thr-NH₂;
H-hArg(hexyl)₂-Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NHEt;
Ac-D-hArg(Et)₂-Gly-Cys-Phe-D-Trp-Lys-Thr-Cys-Phe-NH₂;
Propionyl-D-hArg(Et)₂-Gly-Cys-Phe-D-Trp-Lys(iPr)-Thr-Cys-Thr-NH₂;
Ac-D-β-Nal-Gly-Cys-Phe-D-Trp-Lys-Thr-Cys-Gly-hArg(Et)₂-NH₂;
Ac-D-Lys(iPr)-Gly-Cys-Phe-D-Trp-Lys-Thr-Cys-Thr-NH₂;
Ac-D-hArg(Et)₂-D-hArg(Et)₂-Gly-Cys-Phe-D-Trp-Lys-Thr-Cys-Thr-NH₂;
Ac-Cys-Lys-Asn-4-Cl-Phe-Phe-D-Trp-Lys-Thr-Phe-Thr-Ser-D-Cys-NH₂;
Bmp-Tyr-D-Trp-Lys-Val-Cys-Thr-NH₂;
Bmp-Tyr-D-Trp-Lys-Val-Cys-Phe-NH₂;
Bmp-Tyr-D-Trp-Lys-Val-Cys-p-Cl-Phe-NH₂;
Bmp-Tyr-D-Trp-Lys-Val-Cys-β-Nal-NH₂;
H-D-β-Nal-Cys-Tyr-D-Trp-Lys-Val-Cys-Thr-NH₂;
H-D-Phe-Cys-Tyr-D-Trp-Lys-Abu-Cys-Thr-NH2;
H-D-Phe-Cys-Tyr-D-Trp-Lys-Abu-Cys-β-Nal-NH₂;
H-pentafluoro-D-Phe-Cys-Tyr-D-Trp-Lys-Val-Cys-Thr-NH₂;
Ac-D-β-Nal-Cys-pentafluoro-Phe-D-Trp-Lys-Val-Cys-Thr-NH₂;
H-D-β-Nal-Cys-Tyr-D-Trp-Lys-Val-Cys-β-Nal-NH₂;
H-D-Phe-Cys-Tyr-D-Trp-Lys-Val-Cys-β-Nal-NH₂;
H-D-β-Nal-Cys-Tyr-D-Trp-Lys-Abu-Cys-Thr-NH₂;
H-D-p-Cl-Phe-Cys-Tyr-D-Trp-Lys-Abu-Cys-Thr-NH₂;
Ac-D-p-Cl-Phe-Cys-Tyr-D-Trp-Lys-Abu-Cys-Thr-NH₂;
H-D-Phe-Cys-β-Nal-D-Trp-Lys-Val-Cys-Thr-NH₂;
H-D-Phe-Cys-Tyr-D-Trp-Lys-Cys-Thr-NH₂;
cyclo (Pro-Phe-D-Trp-N-Me-Lys-Thr-Phe);
cyclo (Pro-Phe-D-Trp=Lys-Thr-N-Me-Phe);
cyclo (N-Me-Ala-Tyr-D-Trp-Lys-Thr-Phe);
cyclo (Pro-Tyr-D-Trp-Lys-Thr-Phe);
cyclo (Pro-Phe-D-Trp-Lys-Thr-Phe);
cyclo (Pro-Phe-L-Trp-Lys-Thr-Phe);
cyclo (Pro-Phe-D-Trp(F)-Lys-Thr-Phe);
cyclo (Pro-Phe-Trp(F)-Lys-Thr-Phe);
cyclo (Pro-Phe-D-Trp-Lys-Ser-Phe);
cyclo (Pro-Phe-D-Trp-Lys-Thr-p-Cl-Phe);
cyclo (D-Ala-N-Me-D-Phe-D-Thr-D-Lys-Trp-D-Phe);
cyclo (D-Ala-N-Me-D-Phe-D-Val-Lys-D-Trp-D-Phe);
cyclo (D-Ala-N-Me-D-Phe-D-Thr-Lys-D-Trp-D-Phe);
cyclo (D-Abu-N-Me-D-Phe-D-Val-Lys-D-Trp-D-Tyr);
cyclo (Pro-Tyr-D-Trp-t-4-AchxAla-Thr-Phe);
cyclo (Pro-Phe-D-Trp-t-4-AchxAla-Thr-Phe);
cyclo (N-Me-Ala-Tyr-D-Trp-Lys-Val-Phe);
cyclo (N-Me-Ala-Tyr-D-Trp-t-4-AchxAla-Thr-Phe);
cyclo (Pro-Tyr-D-Trp-4-Amphe-Thr-Phe);
cyclo (Pro-Phe-D-Trp-4-Amphe-Thr-Phe);
cyclo (N-Me-Ala-Tyr-D-Trp-4-Amphe-Thr-Phe);
cyclo (Asn-Phe-Phe-D-Trp-Lys-Thr-Phe-Gaba);
cyclo (Asn-Phe-Phe-D-Trp-Lys-Thr-Phe-Gaba-Gaba);
cyclo (Asn-Phe-D-Trp-Lys-Thr-Phe);
cyclo (Asn-Phe-Phe-D-Trp-Lys-Thr-Phe-NH(CH₂)₄CO);
cyclo (Asn-Phe-Phe-D-Trp-Lys-Thr-Phe-β-Ala);
cyclo (Asn-Phe-Phe-D-Trp-Lys-Thr-Phe-D-Glu)-OH;
cyclo (Phe-Phe-D-Trp-Lys-Thr-Phe);
cyclo (Phe-Phe-D-Trp-Lys-Thr-Phe-Gly);
cyclo (Phe-Phe-D-Trp-Lys-Thr-Phe-Gaba);
cyclo (Asn-Phe-Phe-D-Trp-Lys-Thr-Phe-Gly);
cyclo (Asn-Phe-Phe-D-Trp(F)-Lys-Thr-Phe-Gaba);
cyclo (Asn-Phe-Phe-D-Trp(NO₂)-Lys-Thr-Phe-Gaba);
cyclo (Asn-Phe-Phe-Trp(Br)-Lys-Thr-Phe-Gaba);
cyclo (Asn-Phe-Phe-D-Trp-Lys-Thr-Phe(I)-Gaba);
cyclo (Asn-Phe-Phe-D-Trp-Lys-Thr-Tyr(But)-Gaba);
cyclo (Bmp-Lys-Asn-Phe-Phe-D-Trp-Lys-Thr-Phe-Thr-Pro-Cys)-OH;
cyclo (Bmp-Lys-Asn-Phe-Phe-D-Trp-Lys-Thr-Phe-Thr-Tpo-Cys)-OH;
cyclo (Bmp-Lys-Asn-Phe-Phe-D-Trp-Lys-Thr-Phe-Thr-MeLeu-Cys)-OH;
cyclo (Phe-Phe-D-Trp-Lys-Thr-Phe-Phe-Gaba);
cyclo (Phe-Phe-D-Trp-Lys-Thr-Phe-D-Phe-Gaba;
cyclo (Phe-Phe-D-Trp(5F)-Lys-Thr-Phe-Phe-Gaba);
cyclo (Asn-Phe-Phe-D-Trp-Lys(Ac)-Thr-Phe-NH-(CH₂)₃-CO);
cyclo (Lys-Phe-Phe-D-Trp-Lys-Thr-Phe-Gaba); or
cyclo (Orn-Phe-Phe-D-Trp-Lys-Thr-Phe-Gaba); or
a pharmaceutically acceptable salt thereof.

In one embodiment of the present disclosure, the somatostatin agonist is:
D-β-Nal-Cys-Tyr-D-Trp-Lys-Thr-Cys-Thr-NH₂;
H-Cys-Phe-Phe-D-Trp-Lys-Thr-Phe-Cys-NH₂; 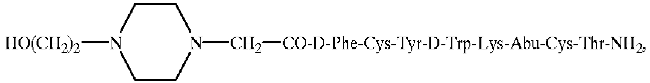 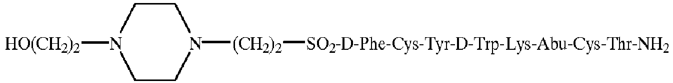
D-Phe-cyclo(Cys-Phe-D-Trp-Lys-Thr-Cys)-Thr-ol; or
a pharmaceutically acceptable salt thereof.

In another embodiment of the present disclosure, the Somatostatin agonist is:
D-Phe-cyclo(Cys-Phe-D-Trp-Lys-Thr-Cys)-Thr-ol or a pharmaceutically acceptable salt thereof.

In one embodiment of the present disclosure, the nucleic acid encodes human GHR. The nucleic acid may have a nucleotide sequence as shown in SEQ ID NO:4 or SEQ ID NO:5.

In one embodiment of the present disclosure, the oligonucleotide is from 12 to 50 nucleobases in length. In another embodiment of the present disclosure, the oligonucleotide is from 15 to 30 nucleobases in length.

In one embodiment of the present disclosure, the oligonucleotide is a DNA oligonucleotide. In another embodiment of the present disclosure, the oligonucleotide is a RNA oligonucleotide, for example, a short interfering RNA (siRNA). In another embodiment of the present disclosure, the oligonucleotide is a chimeric oligonucleotide.

In one embodiment of the present disclosure, the oligonucelotide has at least 70% complementarity with the nucleic acid encoding GHR. In another embodiment of the present disclosure, the oligonucelotide has at least 80% complementarity with the nucleic acid encoding GHR. In another embodiment of the present disclosure, the oligonucelotide has at least 90% complementarity with the nucleic acid encoding GHR. In another embodiment of the present disclosure, the oligonucelotide has at least 95% complementarity, for example, 96%, 97%, 98%, or 99% complementarity with the nucleic acid encoding GHR.

In one embodiment of the present disclosure, the oligonucelotide comprises at least an 8 consecutive nucleobase portion of SEQ ID NO: 6, 7, 8, 9, 10, 11, 12, 13, 14, 15, 16, 17, 18, 19, 21, 22, 23, 24, 25, 26, 27, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 68, 69, 70, 71, 72, 73, 74, 75, 76, 78, 79, 80, or 81.

In another embodiment of the present disclosure, the oligonucelotide consists of the nucelobase sequence of SEQ ID NOs: 6, 7, 8, 9, 10, 11, 12, 13, 14, 15, 16, 17, 18, 19, 21, 22, 23, 24, 25, 26, 27, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 60, 61, 62, 63, 64, 65, 66, 68, 69, 70, 71, 72, 73, 74, 75, 76, 78, 79, 80, or 81.

In one embodiment of the present disclosure, the oligonucleotide consists of the nucleobase sequence of SEQ ID NO:6.

In one embodiment of the present disclosure, the oligonucleotide specifically hybridises with a region encoding GHR, wherein the region comprises a translation initiation codon, a termination codon, a coding region, a 5' untranslated region, a 3' untranslated region, an intron:exon junction or an exon:intron junction. In one embodiment of the present disclosure, the region comprises at least an 8 consecutive nucleobase portion of a sequence selected from SEQ ID NOs: 84-154.

In one embodiment of the present disclosure, the oligonucleotide comprises at least an 8 consecutive nucleobase portion complementary to a region of SEQ ID NO:4 selected from the group consisting of nucleotides 260-339, 332-351 and 344-423 of SEQ ID NO:4.

In one embodiment of the present disclosure, the oligonucleotide inhibits the expression of GHR and/or growth hormone binding protein (GHBP) by at least 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45%.

In one embodiment of the present disclosure, the oligonucleotide comprises at least one modified internucleoside linkage, sugar moiety, or nucleobase. The oligonucleotide may, for example, comprise at least one 2'-O-methoxyethyl sugar moiety and/or at least one phosphorothioate internucleoside linkage and/or at least one 5-methylcytosine.

In one embodiment of the present disclosure, the oligonucleotide consists of 20 linked nucleosides, wherein the oligonucleotide consists of a nucleobase of SEQ ID NO:6; and wherein the oligonucleotide consists of a ten deoxynucleotide region flanked on both the 5' end and the 3' end of said ten deoxynucleotide region with five 2'-O-(2-methoxyethyl) nucleotides, and wherein each internucleoside linkage in the oligonucleotide is a phosphorothioate linkage, and wherein each cytosine in said oligonucleotide is a 5-methylcytosine.

The present disclosure also provides a method of reducing the level of IGF-I in a subject, the method comprising administering a Somatostatin agonist in combination with an oligonucleotide 8 to 80 nucleobases in length targeted to a nucleic acid encoding GHR so as to inhibit expression of the GHR, thereby reducing the level of IGF-I in the subject. In one embodiment of the present disclosure, the level of serum/plasma IGF-I is reduced.

In one embodiment of the present disclosure, the method further comprises identifying a subject in need of a reduction in said subject's GHR and/or IGF-I levels, for example serum/plasma IGF-I levels.

The Somatostatin agonist, the GHR and the oligonucleotide may be further characterized by any one of the above features.

The present disclosure also provides for use of a Somatostatin agonist and an oligonucleotide 8 to 80 nucleobases in length targeted to a nucleic acid encoding GHR in the manufacture of a medicament for the treatment or prevention of a disease caused by and/or associated with an increased level of IGF-I. In one embodiment of the present disclosure, the level of serum/plasma IGF-I is reduced.

In one embodiment of the present disclosure, the disease is acromegaly, gigantism diabetic retinopathy, diabetic nephropathy, or an IGF-I positive cancer such as prostate, myeloma, lung, breast, or colon cancer.

The Somatostatin agonist, the GHR and the oligonucleotide may be further characterized by any one of the above features.

The present disclosure also provides for use of a Somatostatin agonist and an oligonucleotide 8 to 80 nucleobases in length targeted to a nucleic acid encoding GHR in the manufacture of a medicament for reducing the level of IGF-I in a subject.

The Somatostatin agonist, the GHR and the oligonucleotide may be further characterized by any one of the above features.

The present disclosure also provides for a composition comprising a Somatostatin agonist and an oligonucleotide 8 to 80 nucleobases in length targeted to a nucleic acid encoding GHR for the treatment or prevention of a disease caused by and/or associated with an increased level of IGF-I.

The Somatostatin agonist, the GHR and the oligonucleotide may be further characterized by any one of the above features.

The present disclosure also provides for a composition comprising a Somatostatin agonist and an oligonucleotide 8 to 80 nucleobases in length targeted to a nucleic acid encoding GHR for reducing the level of IGF-I in a subject.

The Somatostatin agonist, the GHR and the oligonucleotide may be further characterized by any one of the above features.

Any embodiment of the present disclosure herein shall be taken to apply *mutatis mutandis* to any other embodiment of the present disclosure unless specifically stated otherwise.

The disclosure is hereinafter described by way of the following non-limiting Examples and with reference to the accompanying Figures.

### KEY TO THE SEQUENCE LISTING

**SEQ ID NO:1** - Somatostatin nucleotide sequence
**SEQ ID NO:2** - Somatostatin polypeptide sequence
**SEQ ID NO:3** - Octreotide peptide sequence
**SEQ ID NO:4** - Human growth hormone receptor (hGHR) cDNA sequence
**SEQ ID NO:5** - hGHR gene sequence
**SEQ ID NO:6-83 -** Oligonucleotides targeted to hGHR
**SEQ ID NO:84-154** - Target sequences of hGHR

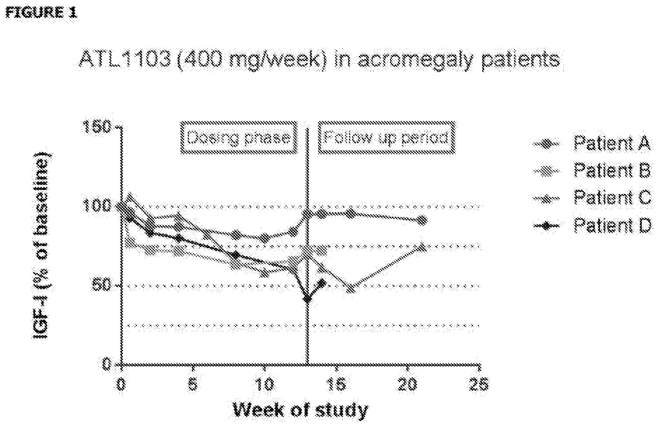

**Figure 1** is a graphical representation of data showing reduction in IGF-1 levels as a percentage of base line with subjects with acromegaly treated with twice weekly 200mg (400mg/week) ATL1103 (SEQ ID No 6) antisense oligonucleotide targeted to the growth hormone receptor (GHR).

### DETAILED DESCRIPTION

The invention is defined by the appended claims. Any embodiment not falling under the scope of the appended claims does not form part of the invention.

### General techniques and selected definitions

Unless specifically defined otherwise, all technical and scientific terms used herein shall be taken to have the same meaning as commonly understood by one of ordinary skill in the art (e.g., in antisense technology, recombinant technology, cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), F.M. Ausubel et al. (editors), Current Protocols in Molecular Biology, Greene Pub. Associates and Wiley-Interscience (1988, including all updates until present), E. Harlow and D. Lane (editors), Antibodies: A Laboratory Manual, Cold Spring Harbour Laboratory (1988), and J.E. Coligan et al. (editors), Current Protocols in Immunology, John Wiley and Sons (including all updates until present).

The term "and/or", for example, "X and/or Y" shall be understood to mean either "X and Y" or "X or Y" and shall be taken to provide explicit support for both meanings or for either meaning.

As used herein, "about" or "approximately" shall generally mean within 20%, more preferably within 10%, and even more preferably within 5%, of a given value or range.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, group of steps or group of compositions of matter.

### Abbreviations

β-Nal = β-naphthylalanine
β-Pal = β-pyridylalanine
hArg(Bu) = N-guanidino-(butyl)-homoarginine
hArg(Et)₂ = N,N'-guanidino-(diethyl)-homoarginine
hArg(CH₂CF₃)₂ = N,N'-guanidino-bis-(2,2,2,-trifluoroethyl)-homoarginine
hArg(CH₃, hexyl) = N,N'-guanidino-(methyl, hexyl)-homoarginine
Lys(Me) = N*^{E}*-methyllysine
Lys(iPr) = N-isopropyllysine
AmPhe = aminomethylphenylalanine
AchxAla = aminocyclohexylalanine
Abu = α-aminobutyric acid
Tpo = 4-thiaproline
MeLeu = N-methylleucine
Orn = ornithine
Nle = norleucine
Nva = norvaline
Trp(Br) = 5-bromo-tryptophan
Trp(F) = 5-fluoro-tryptophan
Trp(NO₂) = 5-nitro-tryptophan
Gaba = γ-aminobutyric acid
Bmp = β-mercaptopropionyl
Ac = acetyl
Pen = pencillamine.

### Treatment and prevention of IGF-I positive diseases

The present disclosure provides methods useful in the prevention and/or treatment of a disease, disorder, or condition caused by insulin-like growth factor I (IGF-I) and/or associated with an increased level of insulin-like growth factor I (IGF-I). As used herein, the term "treatment" refers to administering a pharmaceutical composition to effect an alteration or improvement of a disease, disorder, or condition. As used herein, the term "prevention" refers to administering a pharmaceutical composition to stop or hinder the development of at least one symptom of a disease, disorder, or condition. The subject targeted for treatment is a mammal, preferably a human. As used herein, "an increased level of insulin-like growth factor I (IGF-I)" includes a level above the normal range or in the normal range, for example, at the high end of the normal range, adjusted for age and sex.

The methods involve administration of a Somatostatin analog having Somatostatin receptor agonistic activity and an oligonucleotide targeted to growth hormone receptor (GHR) to a subject. The "subject" can be any mammal, preferably a human. Although not wishing to be limited to the theory, the oligonucleotide acts to inhibit GHR expression in said subject, whilst the Somatostatin agonist acts to prevent production of GH, reducing GH binding to the GHR, thereby reducing the level of IGF-I (which is produced in response to GH signalling) in the subject. Insulin-like growth factor I is a ubiquitous polypeptide important in proliferation, having potent mitogenic effects on a broad range of cells, and important in cell survival, regulating apoptosis on a broad range of cells.

Although not wishing to be limited to theory, the antisense oligonucleotide inhibits GHR expression at the RNA level whilst the Somatostatin agonist may increase GHR or growth hormone binding protein (GHBP) or both. In one embodiment of the present disclosure, the oligonucleotide also acts to reduce GHBP expression. The GHBP is the soluble extracellular portion of the GH receptor, derived by alternative mRNA splicing of the mRNA transcript (in for example, mice and rats) or proteolytic cleavage of the GHR (in for example, humans, cows and pigs). The GHBP may bind GH, increasing the half life of GH, and increasing GH action.

In one embodiment of the present disclosure, the treatment reduces or prevents occurrence of one or more symptoms of acromegaly, for example, reducing the increased serum IGF-I levels in acromegaly to normal levels, or reducing soft tissue swelling, enlargement of internal organs, extremities like overgrowth of the jaw, enlargement of hands and feet, deepening of the voice, thickening of skin, offensive body odor, articular cartilage problems, hyperphosphatemia, peripheral neuropathies, higher blood pressure, diabetes, heart disease, and cancer.

In another embodiment of the present disclosure, the treatment reduces or prevents occurrence of one or more symptoms of retinopathy, for example, reducing new blood vessel formation and/or edema, blurred, double, or distorted vision, or difficulty reading, floaters or spots in vision, loss of vision or a shadow or veil across field of vision, pain, pressure, or constant redness of the eye.

In another embodiment of the present disclosure, the treatment reduces or prevents occurrence of one or more symptoms of diabetic nephropathy, for example glomerula filtration, microalbuminuria, proteinuria, renal damage, swelling in the legs, nausea and vomiting, malaise, fatigue, headache, itching, frequent hiccups, unintended weight loss, swelling of the face, unintended weight gain due to fluid buildup, and high blood pressure.

In another embodiment of the present disclosure, the treatment reduces the size and/or growth of a tumor or cancer (such as prostate, myeloma, lung, breast, or colon cancer) and/or delays progression of the tumor or cancer (such as prostate cancer) from androgen responsive/dependent to androgen unresponsive/independent. Tumor or cancer size and/or growth may be reduced, for example, by reducing the proliferation rate of the tumor/cancer cells, increasing the apoptotic rate of the tumor/cancer cells, modulating tumor/cancer cell signaling, chemosensitization, and/or inhibiting adhesion, anchorage, metastasis of the tumor/cancer cells and/or transformation of cells, for example, prostate cells. The treatment may, for example, reduce IGF-I levels at the high end of the normal range to lower levels, and/or from the top quartile to the 2^{nd} quartile, 3^{rd} or 4^{th} quartile, and/or from the 2^{nd} quartile to the 3^{rd} or 4^{th} quartile as adjusted for age and sex. The treatment may reduce endocrine, autocrine or paracrine levels of IGF-I as antisense oligonucleotides and the Somatostatin agonist may work in the tissues.

### Somatostatin and its agonists

Somatostatin (somatotropin release inhibiting factor or SRIF) has both a 14 amino acid isoform (somatostatin-14) and a 28 amino acid isoform (somatostatin-28) (See Wilson, J. & Foster, D., Williams Textbook of Endocrinology, p. 510 (7th ed., 1985)). The compound is an inhibitor of secretion of the growth hormone and was originally isolated from the hypothalamus (Brazeau et al., 1973). Native somatostatin has a very short duration of effect in vivo since it is rapidly inactivated by endo- and exopeptidase. Many novel analogs have been prepared in order to enhance the duration of effect, biological activity, and selectivity (e.g., for the particular somatostatin receptor) of this hormone. Such analogs will be called "somatostatin agonists" herein. Further, compounds that are short peptides modified by organic moieties and non-peptides, such as organic molecules that do not have an art-recognized amino acid as part of its structure, that bind to somatostatin receptor(s) are also within the meaning of "somatostatin agonists".

Various somatostatin receptors (SSTRs) have been isolated, for example, SSTR-1, SSTR-2, SSTR-3, SSTR-4, and SSTR-5. Thus, the somatostatin agonist may be a SSTR-1 agonist, SSTR-2 agonist, SSTR-3 agonist, SSTR-4 agonist of a SSTR-5 agonist. Preferably, the SSTR agonist has a high affinity (e.g., Ki of less than 1 nM or, preferably, of less than 10 nM) for the SSTR. The somatostatin agonist may also be selective for a particular somatostatin receptor, for example, have a higher binding affinity for a particular somatostatin receptor subtype. Preferably the somatostatin agonist when used in the treatment of acromegaly or gigantism is more selective for SSTR-2, like Octreotide, or Lanreotide, or binds SSTR-1,2,3 and 5 like Pasireotide.

Somatostatin agonists which can be used in the methods of the present disclosure, but are not limited to, those covered by formulae or those specifically recited in the publications set forth below:
EP Application No. 25 164 EU;
Van Binst et al. Peptide Research (1992) 5:8;
Horvath, A. et al. Abstract, "Conformations of Somatostatin Analogs Having Antitumor Activity", 22nd European peptide Symposium, Sep. 13-19, 1992, Interlaken, Switzerland;
PCT Application WO 91/09056 (1991);
EP Application 0 363 589 A2 (1990);
U.S. Pat. No. 4,904,642 (1990);
U.S. Pat. No. 4,871,717 (1989);
U.S. Pat. No. 4,853,371 (1989);
U.S. Pat. No. 4,725,577 (1988);
U.S. Pat. No. 4,684,620 (1987)
U.S. Pat. No. 4,650,787 (1987);
U.S. Pat. No. 4,603,120 (1986);
U.S. Pat. No. 4,585,755 (1986);
EP Application 0 203 031 A2 (1986);
U.S. Pat. No. 4,522,813 (1985);
U.S. Pat. No. 486,415 (1984);
U.S. Pat. No. 4,485,101 (1984);
U.S. Pat. No. 4,435,385 (1984);
U.S. Pat. No. 4,395,403 (1983);
U.S. Pat. No. 4,369,179 (1983);
U.S. Pat. No. 4,360,516 (1982);
U.S. Pat. No. 4,358,439 (1982);
U.S. Pat. No. 4,328,214 (1982);
U.S. Pat. No. 4,316,890 (1982);
U.S. Pat. No. 4,310,518 (1982);
U.S. Pat. No. 4,291,022 (1981);
U.S. Pat. No. 4,238,481 (1980);
U.S. Pat. No. 4,235,886 (1980);
U.S. Pat. No. 4,224,190 (1980);
U.S. Pat. No. 4,211,693 (1980);
U.S. Pat. No. 4,190,648 (1980);
U.S. Pat. No. 4,146,612 (1979); and
U.S. Pat. No. 4,133,782 (1979).

Examples of somatostatin agonists include, but are not limited to, the following somatostatin analogs and pharmaceutically acceptable salts thereof which are disclosed in the above-cited references:
D-β-Nal-Cys-Tyr-D-Trp-Lys-Thr-Cys-Thr-NH₂;
D-Phe-Cys-Phe-D-Trp-Lys-Thr-Cys-β-Nal-NH₂;
D-Phe-Cys-Tyr-D-Trp-Lys-Thr-Cys-β-Nal-NH₂;
D-β-Nal-Cys-Phe-D-Trp-Lys-Thr-Cys-Thr-NH₂;
D-Phe-Cys-Tyr-D-Trp-Lys-Thr-Pen-Thr-NH₂;
D-Phe-Cys-Phe-D-Trp-Lys-Thr-Pen-Thr-NH₂;
D-Phe-Cys-Tyr-D-Trp-Lys-Thr-Pen-Thr-OH;
D-Phe-Cys-Phe-D-Trp-Lys-Thr-Pen-Thr-OH;
Gly-Pen-Phe-D-Trp-Lys-Thr-Cys-Thr-OH;
Phe-Pen-Tyr-D-Trp-Lys-Thr-Cys-Thr-OH;
Phe-Pen-Phe-D-Trp-Lys-Thr-Pen-Thr-OH;
H-D-Phe-Cys-Phe-D-Trp-Lys-Thr-Cys-Thr-ol;
H-D-Phe-Cys-Phe-D-Trp-Lys-Thr-Cys-Thr-NH₂;
H-D-Trp-Cys-Tyr-D-Trp-Lys-Val-Cys-Thr-NH₂;
H-D-Trp-Cys-Phe-D-Trp-Lys-Thr-Cys-Thr-NH₂;
H-D-Phe-Cys-Tyr-D-Trp-Lys-Val-Cys-Thr-NH₂;
H-D-Phe-Cys-Tyr-D-Trp-Lys-Val-Cys-Trp-NH₂;
H-D-Phe-Cys-Tyr-D-Trp-Lys-Val-Cys-Thr-NH₂;
Ac-D-Phe-Lys*-Tyr-D-Trp-Lys-Val-Asp-Thr-NH₂ (an amide bridge formed between Lys* and Asp);
Ac-hArg(Et)₂Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NH₂;
Ac-D-hArg(BU)-Gly-Cys-Phe-D-Trp-Lys-Thr-Cys-Thr-NH₂;
Ac-D-hArg(Et)₂-Cys-Phe-D-Trp-Lys-Thr-Cys-Thr-NH₂;
Ac-L-hArg(Et)₂-Cys-Phe-D-Trp-Lys-Thr-Cys-Thr-NH₂;
Ac-D-hArg(CH₂CF₃)₂-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys-Thr-Cys-Phe-NH₂;
Ac-D-hArg(CH₂CF₃)₂-Gly-Cys-Phe-D-Trp-Lys-Thr-Cys-Thr-NHEt;
Ac-L-hArg(CH₂CF₃)₂-Gly-Cys-Phe-D-Trp-Lys-Thr-Cys-Thr-NH₂;
Ac-D-hArg(CH₂CF₃)₂-Gly-Cys-Phe-D-Trp-Lys(Me)-Thr-Cys-Thr-NH₂;
Ac-D-hArg(CH₂CF₃)₂-Gly-Cys-Phe-D-Trp-Lys(Me)-Thr-Cys-Thr-NHEt;
Ac-hArg(CH3, hexyl)-Gly-Cys-Phe-D-Trp-Lys-Thr-Cys-Thr-NH₂;
H-hArg(hexyl)₂-Gly-Cys-Phe-D-Trp-Lys-Thr-Cys-Thr-NH₂;
Ac-D-hArg(Et)₂-Gly-Cys-Phe-D-Trp-Lys-Thr-Cys-Thr-NHEt;
Ac-D-hArg(Et)₂-Gly-Cys-Phe-D-Trp-Lys-Thr-Cys-Phe-NH₂;
Propionyl-D-hArg(Et)₂-Gly-Cys-Phe-D-Trp-Lys(iPr)-Thr-Cys-Thr-NH₂;
Ac-D-β-Nal-Gly-Cys-Phe-D-Trp-Lys-Thr-Cys-Gly-hArg(Et)₂-NH₂;
Ac-D-Lys(iPr)-Gly-Cys-Phe-D-Trp-Lys-Thr-Cys-Thr-NH₂;
Ac-D-hArg(CH₂CF₃)₂-D-hArg(CH₂CF₃)₂-Gly-Cys-Phe-D-Trp-Lys-Thr-Cys-Thr-NH₂;
Ac-D-hArg(CH₂CF₃)₂-D-hArg(CH₂CF₃)₂-Gly-Cys-Phe-D-Trp-Lys-Thr-Cys-Phe-NH₂;
Ac-D-hArg(Et)₂-D-hArg(Et)₂-Gly-Cys-Phe-D-Trp-Lys-Thr-Cys-Thr-NH₂;
Ac-Cys-Lys-Asn-4-Cl-Phe-Phe-D-Trp-Lys-Thr-Phe-Thr-Ser-D-Cys-NH₂;
Bmp-Tyr-D-Trp-Lys-Val-Cys-Thr-NH₂;
Bmp-Tyr-D-Trp-Lys-Val-Cys-Phe-NH₂;
Bmp-Tyr-D-Trp-Lys-Val-Cys-p-Cl-Phe-NH₂;
Bmp-Tyr-D-Trp-Lys-Val-Cys-β-Nal-NH₂;
H-D-β-Nal-Cys-Tyr-D-Trp-Lys-Val-Cys-Thr-NH₂;
H-D-Phe-Cys-Tyr-D-Trp-Lys-Abu-Cys-Thr-NH₂;
H-D-Phe-Cys-Tyr-D-Trp-Lys-Abu-Cys-β-Nal-NH₂;
H-pentafluoro-D-Phe-Cys-Tyr-D-Trp-Lys-Val-Cys-Thr-NH₂;
Ac-D-β-Nal-Cys-pentafluoro-Phe-D-Trp-Lys-Val-Cys-Thr-NH₂;
H-D-β-Nal-Cys-Tyr-D-Trp-Lys-Val-Cys-β-Nal-NH₂;
H-D-Phe-Cys-Tyr-D-Trp-Lys-Val-Cys-β-Nal-NH₂;
H-D-β-Nal-Cys-Tyr-D-Trp-Lys-Abu-Cys-Thr-NH₂;
H-D-p-Cl-Phe-Cys-Tyr-D-Trp-Lys-Abu-Cys-Thr-NH₂;
Ac-D-p-Cl-Phe-Cys-Tyr-D-Trp-Lys-Abu-Cys-Thr-NH₂;
H-D-Phe-Cys-β-Nal-D-Trp-Lys-Val-Cys-Thr-NH₂;
H-D-Phe-Cys-Tyr-D-Trp-Lys-Cys-Thr-NH₂;
cyclo (Pro-Phe-D-Trp-N-Me-Lys-Thr-Phe);
cyclo (Pro-Phe-D-Trp-Lys-Thr-N-Me-Phe);
cyclo (N-Me-Ala-Tyr-D-Trp-Lys-Thr-Phe);
cyclo (Pro-Tyr-D-Trp-Lys-Thr-Phe);
cyclo (Pro-Phe-D-Trp-Lys-Thr-Phe);
cyclo (Pro-Phe-L-Trp-Lys-Thr-Phe);
cyclo (Pro-Phe-D-Trp(F)-Lys-Thr-Phe);
cyclo (Pro-Phe-D-Trp-Lys-Ser-Phe);
cyclo (Pro-Phe-D-Trp-Lys-Thr-p-Cl-Phe);
cyclo (D-Ala-N-Me-D-Phe-D-Thr-D-Lys-Trp-D-Phe);
cyclo (D-Ala-N-Me-D-Phe-D-Val-Lys-D-Trp-D-Phe);
cyclo (D-Ala-N-Me-D-Phe-D-Thr-Lys-D-Trp-D-Phe);
cyclo (D-Abu-N-Me-D-Phe-D-Val-Lys-D-Trp-D-Tyr);
cyclo (Pro-Tyr-D-Trp-t-4-AchxAla-Thr-Phe);
cyclo (Pro-Phe-D-Trp-t-4-AchxAla-Thr-Phe);
cyclo (N-Me-Ala-Tyr-D-Trp-Lys-Val-Phe);
cyclo (N-Me-Ala-Tyr-D-Trp-t-4-AchxAla-Thr-Phe);
cyclo (Pro-Tyr-D-Trp-4-Amphe-Thr-Phe);
cyclo (Pro-Phe-D-Trp-4-Amphe-Thr-Phe);
cyclo (N-Me-Ala-Tyr-D-Trp-4-Amphe-Thr-Phe);
cyclo (Asn-Phe-Phe-D-Trp-Lys-Thr-Phe-Gaba);
cyclo (Asn-Phe-Phe-D-Trp-Lys-Thr-Phe-Gaba-Gaba);
cyclo (Asn-Phe-D-Trp-Lys-Thr-Phe);
cyclo (Asn-Phe-D-Trp-Lys-Thr-Lys-Thr-Phe-NH(CH₂)₄CO);
cyclo (Asn-Phe-Phe-D-Trp-Lys-Thr-Phe-β-Ala;
cyclo (Asn-Phe-Phe-D-Trp-Lys-Thr-Phe-D-Glu)-OH;
cyclo (Phe-Phe-D-Trp-Lys-Thr-Phe);
cyclo Phe-Phe-D-Trp-Lys-Thr-Phe-Gly);
cyclo (Phe-Phe-D-Trp-Lys-Thr-Phe-Gaba);
cyclo (Asn-Phe-Phe-D-Trp-Lys-Thr-Phe-Gly);
cyclo (Asn-Phe-Phe-D-Trp(F)-Lys-Thr-Phe-Gaba);
cyclo (Asn-Phe-Phe-D-Trp(NO₂)-Lys-Thr-Phe-Gaba)
cyclo (Asn-Phe-Phe-Trp(Br)-Lys-Thr-Phe-Gaba);
cyclo (Asn-Phe-Phe-D-Trp-Lys-Thr-Phe(I)-Gaba);
cyclo (Asn-Phe-Phe-D-Trp-Lys-Thr-Tyr(But)-Gaba);
cyclo (Bmp-Lys-Asn-Phe-Phe-D-Trp-Lys-Thr-Phe-Thr-Pro-Cys)-OH;
cyclo (Bmp-Lys-Asn-Phe-Phe-D-Trp-Lys-Thr-Phe-Thr-Tpo-Cys)-OH;
cyclo (Bmp-Lys-Asn-Phe-Phe-D-Trp-Lys-Thr-Phe-Thr-MeLeu-Cys)-OH;
cyclo (Phe-Phe-D-Trp-Lys-Thr-Phe-Phe-Gaba);
cyclo (Phe-Phe-D-Trp-Lys-Thr-Phe-D-Phe-Gaba);
cyclo (Phe-Phe-D-Trp(5F)-Lys-Thr-Phe-Phe-Gaba);
cyclo (Asn-Phe-Phe-D-Trp-Lys(Ac)-Thr-Phe-NH-(CH₂)₃-CO);
cyclo (Lys-Phe-Phe-D-Trp-Lys-Thr-Phe-Gaba);
cyclo (Orn-Phe-Phe-D-Trp-Lys-Thr-Phe-Gaba); and
H-Cys-Phe-Phe-D-Trp-Lys-Thr-Phe-Cys-NH₂.

Note that for all somatostatin agonists described herein, each amino acid residue represents the structure of -NH-C(R)H-CO-, in which R is the side chain (e.g., CH₃ for Ala) except for Thr-ol which means -NH-CH(CH(CH₃)OH)-CH₂-OH and Pro which means prolinyl. Lines between amino acid residues represent peptide bonds which join the amino acids. Also, where the amino acid residue is optically active, it is the L-form configuration icon that is intended unless D-form is expressly designated. A disulfide bridge is formed between two Cys residues; however, it is not shown.

Use of linear somatostatin agonists of the following formula is also within the disclosure: 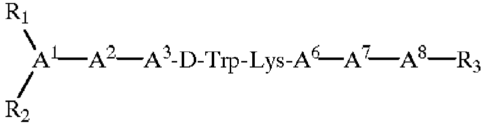 or a pharmaceutically acceptable salt thereof, wherein
A¹ is a D- or L-isomer of Ala, Leu, Ile, Val, Nle, Thr, Ser, β-Nal, β-Pal, Trp, Phe, 2,4-dichloro-Phe, pentafluoro-Phe, p-X-Phe, or o-X-Phe;
A² is Ala, Leu, Ile, Val, Nle, Phe, β-Nal, pyridyl-Ala, Trp, 2,4-dichloro-Phe, pentafluoro-Phe, o-X-Phe, or p-X-Phe;
A³ is pyridyl-Ala, Trp, Phe, β-Nal, 2,4-dichloro-Phe, pentafluoro-Phe, o-X-Phe, or p-X-Phe;
A⁶ is Val, Ala, Leu, Ile, Nle, Thr, Abu, or Ser;
A⁷ is Ala, Leu, Ile, Val, Nle, Phe, β-Nal, pyridyl-Ala, Trp, 2,4-dichloro-Phe, pentafluoro-Phe, o-X-Phe, or p-X-Phe;
A⁸ is a D- or L-isomer of Ala, Leu, Ile, Val, Nle, Thr, Ser, Phe, β-Nal, pyridyl-Ala, Trp, 2,4-dichloro-Phe, pentafluoro-Phe, p-X-Phe, or o-X-Phe;
wherein X for each occurrence is independently selected from the group consisting of CH₃, Cl, Br, F, OH, OCH₃ and NO₂;
each R₁ and R₂, independently, is H, lower acyl or lower alkyl; and R₃ is OH or NH2; provided that at least one of A¹ and A⁸ and one of A² and A⁷ must be an aromatic amino acid; and further provided that A¹, A², A⁷ and A⁸ cannot all be aromatic amino acids.

Examples of linear agonists to be used in the method of this disclosure include:
H-D-Phe-p-chloro-Phe-Tyr-D-Trp-Lys-Thr-Phe-Thr-NH₂;
H-D-Phe-p-NO2-Phe-Tyr-D-Trp-Lys-Val-Phe-Thr-NH₂;
H-D-Nal-p-chloro-Phe-Tyr-D-Trp-Lys-Val-Phe-Thr-NH₂;
H-D-Phe-Phe-Phe-D-Trp-Lys-Thr-Phe-Thr-NH₂;
H-D-Phe-Phe-Tyr-D-Trp-Lys-Val-Phe-Thr-NH₂;
H-D-Phe-p-chloro-Phe-Tyr-D-Trp-Lys-Val-Phe-Thr-NH₂; and
H-D-Phe-Ala-Tyr-D-Trp-Lys-Val-Val-Ala-β-D-Nal-NH₂; or a pharmaceutically acceptable salt thereof.

If desired, one or more chemical moieties, for exmaple, a sugar derivative, mono- or poly-hydroxy C2-12 alkyl, mono or poly-hydroxy C2-12 acyl groups, or a piperazine derivative, can be attached to the somatostatin agonist, e.g., to the N-terminus amino acid. See PCT Application WO 88/02756, European Application 0 329 295, and PCT Application No. WO 94/04752. An example of a somatostatin agonists which contain N-terminal chemical substitutions are: 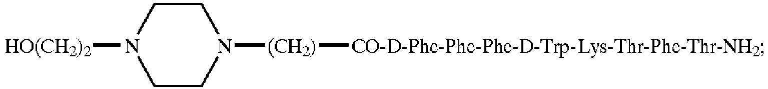 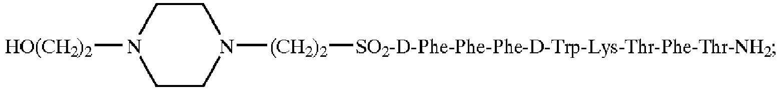 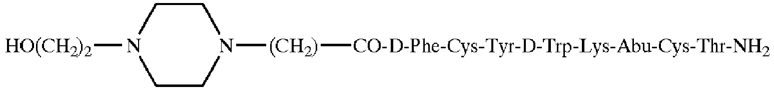 (BIM-23190); and 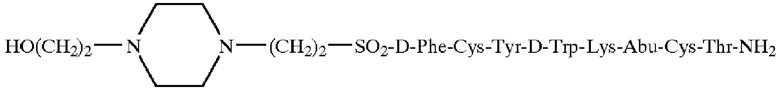 or a pharmaceutically acceptable salt thereof.

In one embodiment of the present disclosure, the Somatostatin agonist is for example Octreotide, Lanreotide, or Pasireotide. In a further embodiment of the present disclosure, the Somatostatin agonist is a long acting release (LAR) form, for example, Octreotide-LAR (Sandostatin™-LAR), Lanreotide-LAR (Lanreotide Autogel), or Pasireotide-LAR, or is Lanreotide microparticles.

### Synthesis of Somatostatin agonists

The methods for synthesizing somatostatin agonists are well documented and are within the ability of a person of ordinary skill in the art, for example, as illustrated in the U.S. Patents and other references cited hereinabove.

Synthesis of short amino acid sequences is well established in the peptide art. For example, synthesis of D-Nal-Cys-Tyr-D-Trp-Lys-Val-Cys-Thr-NH₂, described above, can be synthesized by following the protocol set forth in U.S. Pat. No. 4,853,371 and synthesis of H-D-Phe-Phe-Phe-D-Trp-Lys-Thr-Phe-Thr-NH₂, described above, can be achieved by following the protocol set forth in example I of European Patent Application 0 395 417 A1. The synthesis of somatostatin agonists with a substituted N-terminus can be achieved, for example, by following the protocol set forth in WO 88/02756, European Pat. Application No. 0 329 295, and PCT Publication No. WO 94/04752.

### Conjugates

Somatostatin agonists useful in the methods of the present disclosure may be covalently attached (hereinafter "conjugated") to one or more chemical groups. Such conjugation produces a Somatostatin agonist conjugate having a greater actual molecular weight than the unmodified Somatostatin agonist.

A variety of methods for pegylating proteins have been described (see, for example, US 4,179,337), disclosing the conjugation of a number of hormones and enzymes to PEG and polypropylene glycol to produce physiologically active non-immunogenic compositions. Generally, a PEG having at least one terminal hydroxy group is reacted with a coupling agent to form an activated PEG having a terminal reactive group. This reactive group can then react with the α- and ε-amines of proteins to form a covalent bond. Conveniently, the other end of the PEG molecule can be "blocked" with a non-reactive chemical group, such as a methoxy group, to reduce the formation of PEG-crosslinked complexes of protein molecules.

A composition containing a pegylated Somatostatin agonist for use in a therapeutic formulation can be heterogeneous or homogeneous, i.e., containing multiple or single pegylated Somatostatin agonists. Typically, the composition contains at least 70% one or two forms of pegylated Somatostatin agonists; preferably, at least 80% one or two forms; and more preferably, at least 90% one or two forms.

### Antisense compounds to Growth Hormone Receptor

The methods of the present disclosure rely on the use of an antisense compound to growth hormone receptor (GHR) to modulate growth hormone (GH) signalling or the GH/insulin-like growth factor-I (IGF-I) axis, particularly the expression of GHR and/or IGF-I. Preferably, the antisense compound is an oligonucleotide. However, other oligomeric antisense compounds, including but not limited to oligonucleotide mimetics are contemplated.

Hybridization of an antisense compound with its target nucleic acid is generally referred to as "antisense". Hybridization of the antisense compound with its target nucleic acid inhibits the function of the target nucleic acid. Such "antisense inhibition" is typically based upon hydrogen bonding-based hybridization of the antisense compound to the target nucleic acid such that the target nucleic acid is cleaved, degraded, or otherwise rendered inoperable. The functions of target DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA.

"Hybridization" as used herein means pairing of complementary bases of the oligonucleotide and target nucleic acid. Base pairing typically involves hydrogen bonding, which may be Watson-Crick, Hoogsteen or reversed Hoogsteen hydrogen bonding, between complementary nucleoside or nucleotide bases (nucleobases). Guanine (G) and cytosine (C) are examples of complementary nucleobases which pair through the formation of 3 hydrogen bonds. Adenine (A) and thymine (T) are examples of complementary nucleobases which pair through the formation of 2 hydrogen bonds. Hybridization can occur under varying circumstances.

A "nucleoside" is a base-sugar combination. The base portion of the nucleoside is normally a heterocyclic base. The two most common classes of such heterocyclic bases are the purines and the pyrimidines. "Nucleotides" are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar.

"Specifically hybridizable" and "complementary" are terms which are used to indicate a sufficient degree of complementarity such that stable and specific binding occurs between the antisense compound and target nucleic acid. It is understood that the antisense compound need not be 100% complementary to its target nucleic acid sequence to be specifically hybridizable. An antisense compound is specifically hybridizable when binding of the antisense compound to the target nucleic acid interferes with the expression of the target nucleic acid and there is a sufficient degree of complementarity to avoid non-specific binding of the antisense compound to non-target sequences under conditions in which specific binding is desired, for example, under physiological conditions in the case of therapeutic treatment.

The term "stringent hybridization conditions" or "stringent conditions" as used herein refers to conditions under which the antisense compound will hybridize to its target sequence, but to a minimal number of other sequences. Stringent conditions are sequence-dependent and will be different in different circumstances. Stringent condition under which the antisense compound hybridizes to a target sequence is determined by the nature and composition of the antisense compound and the assays in which it is being investigated.

"Complementary" as used herein, refers to the capacity for precise pairing between a nucleobase of the antisense compound and the target nucleic acid. For example, if a nucleobase at a certain position of the antisense compound is capable of hydrogen bonding with a nucleobase at a certain position of the target nucleic acid, then the position of hydrogen bonding between the antisense compound and the target nucleic acid is considered to be a complementary position. The antisense compound may hybridize over one or more segments, such that intervening or adjacent segments are not involved in the hybridization event (e.g., a loop structure or hairpin structure). In one embodiment of the present disclosure, the antisense compound comprises at least 70% sequence complementarity to a target region within the target nucleic acid. For example, an antisense compound in which 18 of 20 nucleobases are complementary to a target region within the target nucleic acid, and would therefore specifically hybridize, would represent 90% complementarity. In this example, the remaining noncomplementary nucleobases may be clustered or interspersed with complementary nucleobases and need not be contiguous to each other, or to complementary nucleobases. As such, an antisense compound which is 18 nucleobases in length having 4 non-complementary nucleobases which are flanked by 2 regions of complete complementarity with the target nucleic acid would have 77.8% overall complementarity with the target nucleic acid and would thus, fall within the scope of the present disclosure. Percent complementarity of an antisense compound with a region of a target nucleic acid can be determined routinely using BLAST programs (basic local alignment search tools) and PowerBLAST programs known in the art (Altschul *et al.*, 1990; Zhang and Madden, 1997).

### Antisense oligonucleotides

The present disclosure provides for use of an antisense oligonucleotide for inhibiting expression of a growth hormone receptor (GHR).

The term "inhibits" as used herein means any measurable decrease (e.g., 10%, 20%, 50%, 90%, or 100%) in GHR expression.

As used herein, the term "oligonucleotide" refers to an oligomer or polymer of RNA or DNA or mimetics, chimeras, analogs and homologs thereof. This term includes oligonucleotides composed of naturally occurring nucleobases, sugars and covalent internucleoside (backbone) linkages, as well as oligonucleotides having non-naturally occurring portions which function similarly. Such modified or substituted oligonucleotides are often preferred over native forms because of desirable properties such as, for example, enhanced cellular uptake, enhanced affinity for the target nucleic acid and increased stability in the presence of nucleases.

In forming oligonucleotides, phosphate groups covalently link adjacent nucleosides to one another to form a linear polymeric compound. In turn, the respective ends of this linear polymeric compound can be further joined to form a circular compound; however, linear compounds are generally preferred. In addition, linear compounds may have internal nucleobase complementarity and may therefore fold in a manner so as to produce a fully or partially double-stranded compound. With regard to oligonucleotides, the phosphate groups are commonly referred to as forming the internucleoside backbone of the oligonucleotide. The normal linkage or backbone of RNA and DNA is a 3' to 5' phosphodiester linkage.

Antisense oligonucleotides useful in the methods of the present disclosure include, for example, ribozymes, siRNA, external guide sequence (EGS) oligonucleotides, alternate splicers, primers, probes, and other oligonucleotides which hybridize to at least a portion of the target nucleic acid.

Antisense oligonucleotides may be administered in the form of single-stranded, double-stranded, circular or hairpin and may contain structural elements such as internal or terminal bulges or loops. Once administered, the antisense oligonucleotides may elicit the action of one or more enzymes or structural proteins to effect modification of the target nucleic acid.

One non-limiting example of such an enzyme is RNAse H, a cellular endonuclease which cleaves the RNA strand of an RNA:DNA duplex. It is known in the art that single-stranded antisense compounds which are "DNA-like" elicit RNAse H. Activation of RNase H therefore results in cleavage of the RNA target, thereby greatly enhancing the efficiency of oligonucleotide-mediated inhibition of gene expression. Similar roles have been postulated for other ribonucleases, such as those in the RNase III and ribonuclease L family of enzymes.

The introduction of double-stranded RNA (dsRNA) molecules, has been shown to induce potent and specific antisense-mediated reduction of the function of a gene or its associated gene products. This phenomenon occurs in both plants and animals and is believed to have an evolutionary connection to viral defense and transposon silencing.

The first evidence that dsRNA could lead to gene silencing in animals came in 1995 from work in the nematode, *Caenorhabditis elegans* (Guo and Kempheus, 1995). Montgomery *et al.* (1998) have shown that the primary interference effects of dsRNA are posttranscriptional. The posttranscriptional antisense mechanism defined in *Caenorhabditis elegans* resulting from exposure to double-stranded RNA (dsRNA) has since been designated RNA interference (RNAi). This term has been generalized to mean antisense-mediated gene silencing involving the introduction of dsRNA leading to the sequence-specific reduction of endogenous targeted mRNA levels (Fire *et al.*, 1998). It has been shown that it is, in fact, the single-stranded RNA oligomers of antisense polarity of the dsRNAs which are the potent inducers of RNAi (Tijsterman *et al.*, 2002).

A person having ordinary skill in the art could, without undue experimentation, identify antisense oligonucleotides useful in the methods of the present disclosure.

### Modified internucleoside linkages (backbones)

Antisense compounds useful in the methods of the present disclosure include oligonucleotides having modified backbones or non-natural internucleoside linkages. Oligonucleotides having modified backbones include those that retain a phosphorus atom in the backbone and those that do not have a phosphorus atom in the backbone.

Modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates, and boranophosphates having normal 3'-5' linkages, 2'-5' linked analogs of these, and those having inverted polarity wherein one or more internucleotide linkages is a 3' to 3', 5' to 5' or 2' to 2' linkage.

Oligonucleotides having inverted polarity comprise a single 3' to 3' linkage at the 3'-most internucleotide linkage, that is, a single inverted nucleoside residue which may be abasic (the nucleobase is missing or has a hydroxyl group in place thereof). Various salts, mixed salts and free acid forms are also included.

Representative United States patents that teach the preparation of the above phosphorus-containing linkages include, but are not limited to, US 3,687,808, US 4,469,863, US 4,476,301, US 5,023,243, US 5,177,196, US 5,188,897, US 5,264,423, US 5,276,019, US 5,278,302, US 5,286,717, US 5,321,131, US 5,399,676, US 5,405,939, US 5,453,496, US 5,455,233, US 5,466,677, US 5,476,925, US 5,519,126, US 5,536,821, US 5,541,306, US 5,550,111, US 5,563,253, US 5,571,799, US 5,587,361, US 5,194,599, US 5,565,555, US 5,527,899, US 5,721,218, US 5,672,697 and US 5,625,050.

Modified oligonucleotide backbones that do not include a phosphorus atom therein include, for example, backbones formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

Representative United States patents that teach the preparation of the above oligonucleotides include, but are not limited to, US 5,034,506, US 5,166,315, US 5,185,444, US 5,214,134, US 5,216,141, US 5,235,033, US 5,264,562, US 5,264,564, US 5,405,938, US 5,434,257, US 5,466,677, US 5,470,967, US 5,489,677, US 5,541,307, US 5,561,225, US 5,596,086, US 5,602,240, US 5,610,289, US 5,602,240, US 5,608,046, US 5,610,289, US 5,618,704, US 5,623,070, US 5,663,312, US 5,633,360, US 5,677,437, US 5,792,608, US 5,646,269 and US 5,677,439.

### Modified sugar and internucleoside linkages

Antisense compounds useful in the methods of the present disclosure include oligonucleotide mimetics where both the sugar and the internucleoside linkage (i.e. the backbone) of the nucleotide units are replaced with novel groups. The nucleobase units are maintained for hybridization with the target nucleic acid.

An oligonucleotide mimetic that has been shown to have excellent hybridization properties is referred to as a peptide nucleic acid (PNA). In PNA compounds, the sugar-backbone of an oligonucleotide is replaced with an amide containing backbone, in particular, an aminoethylglycine backbone. The nucleobases are retained and are bound directly or indirectly to *aza* nitrogen atoms of the amide portion of the backbone. Representative United States patents that teach the preparation of PNA compounds include, but are not limited to, US 5,539,082, US 5,714,331, and US 5,719,262. Further teaching of PNA compounds can be found in Nielsen *et al.*, 1991.

The antisense compounds useful in the methods of the present disclosure also include oligonucleotides with phosphorothioate backbones and oligonucleotides with heteroatom backbones, for example, -CH₂-NH-O-CH₂-, -CH₂-N(CH₃)-O-CH₂- [known as a methylene (methylimino) or MMI backbone], -CH₂-O-N(CH₃)-CH₂-, -CH₂-N(CH₃)-N(CH₃)-CH₂- and -O-N(CH₃)-CH₂-CH₂- [wherein the native phosphodiester backbone is represented as -O-P-O-CH₂-] of US 5,489,677, and the amide backbones of US 5,602,240.

The antisense compounds useful in the methods of the present disclosure also include oligonucleotides having morpholino backbone structures of US 5,034,506.

### Modified sugars

Antisense compounds useful in the methods of the present disclosure include oligonucleotides having one or more substituted sugar moieties.

Examples include oligonucleotides comprising one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl.

In one embodiment of the present disclosure, the oligonucleotide comprises one of the following at the 2' position: O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃]₂, where n and m are from 1 to about 10.

Further examples include of modified oligonucleotides include oligonucleotides comprising one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, CI, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties.

In one embodiment of the present disclosure, the modification includes 2'-methoxyethoxy (2'-O-CH₂CH₂OCH₃ (also known as 2'-O-(2-methoxyethyl) or 2'-MOE) (Martin *et al.*, 1995), that is, an alkoxyalkoxy group. In a further embodiment of the present disclosure, the modification includes 2'-dimethylaminooxyethoxy, that is, a O(CH₂)₂ON(CH₃)₂ group (also known as 2'-DMAOE), or 2'-dimethylaminoethoxyethoxy (also known in the art as 2'-O-dimethyl-amino-ethoxyethyl or 2'-DMAEOE), that is, 2'-O-CH₂-O-CH₂-N(CH₃)₂.

Other modifications include 2'-methoxy (2'-O-CH₃), 2'-aminopropoxy (2'-OCH₂CH₂CH₂NH₂), 2'-allyl (2'-CH₂-CH=CH₂), 2'-O-allyl (2'-O-CH₂-CH=CH₂) and 2'-fluoro (2'-F). The 2'-modification may be in the arabino (up) position or ribo (down) position. In one embodiment of the present disclosure, a 2'-arabino modification is 2'-F.

Similar modifications may also be made at other positions on the oligonucleotide, particularly the 3' position of the sugar on the 3' terminal nucleotide or in 2'-5' linked oligonucleotides and the 5' position of the 5' terminal nucleotide.

Oligonucleotides may also have sugar mimetics, such as cyclobutyl moieties in place of the pentofuranosyl sugar.

Representative United States patents that teach the preparation of such modified sugar structures include, but are not limited to, US 4,981,957, US 5,118,800, US 5,319,080, US 5,359,044, US 5,393,878, US 5,446,137, US 5,466,786, US 5,514,785, US 5,519,134, US 5,567,811, US 5,576,427, US 5,591,722, US 5,597,909, US 5,610,300, US 5,627,053, US 5,639,873, US 5,646,265, US 5,658,873, US 5,670,633, US 5,792,747, and US 5,700,920.

A further modification of the sugar includes Locked Nucleic Acids (LNAs) in which the 2'-hydroxyl group is linked to the 3' or 4' carbon atom of the sugar ring, thereby forming a bicyclic sugar moiety. In one embodiment of the present disclosure, the linkage is a methylene (-CH₂-)ₙ group bridging the 2' oxygen atom and the 4' carbon atom, wherein n is 1 or 2. LNAs and preparation thereof are described in WO 98/39352 and WO 99/14226.

### Natural and modified nucleobases

Antisense compounds useful in the methods of the present disclosure include oligonucleotides having nucleobase modifications or substitutions. As used herein, "unmodified" or "natural" nucleobases include the purine bases adenine (A) and guanine (G), and the pyrimidine bases thymine (T), cytosine (C) and uracil (U).

Modified nucleobases include other synthetic and natural nucleobases such as, for example, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine.

Further modified nucleobases include tricyclic pyrimidines, such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as, for example, a substituted phenoxazine cytidine (e.g., 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one).

Modified nucleobases may also include those in which the purine or pyrimidine base is replaced with other heterocycles, for example, 7-deaza-adenine, 7-deazaguanosine, 2-aminopyridine and 2-pyridone. Further nucleobases include those disclosed in US 3,687,808, those disclosed in J.I. Kroschwitz (editor), The Concise Encyclopedia of Polymer Science and Engineering, pages 858-859, John Wiley and Sons (1990), those disclosed by Englisch et al. (1991), and those disclosed by Y.S. Sanghvi, Chapter 15: Antisense Research and Applications, pages 289-302, S.T. Crooke, B. Lebleu (editors), CRC Press, 1993.

Certain of these nucleobases are particularly useful for increasing the binding affinity of the oligonucleotide. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C. In one embodiment of the present disclosure, these nucleobase substitutions are combined with 2'-O-methoxyethyl sugar modifications.

Representative United States patents that teach the preparation of certain of the above noted modified nucleobases as well as other modified nucleobases include, but are not limited to, US 3,687,808, US 4,845,205, US 5,130,302, US 5,134,066, US 5,175,273, US 5,367,066, US 5,432,272, US 5,457,187, US 5,459,255, US 5,484,908, US 5,502,177, US 5,525,711, US 5,552,540, US 5,587,469, US 5,594,121, US 5,596,091, US 5,614,617, US 5,645,985, US 5,830,653, US 5,763,588, US 6,005,096, US 5,681,941 and US 5,750,692.

### Conjugates

Antisense compounds useful in the methods of the present disclosure may be conjugated to one or more moieties or groups which enhance the activity, cellular distribution or cellular uptake of the antisense compound.

These moieties or groups may be covalently bound to functional groups such as primary or secondary hydroxyl groups.

Exemplary moieties or groups include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugate groups include cholesterols, lipids, phospholipids, biotin, phenazine, folate, phenanthridine, anthraquinone, acridine, fluoresceins, rhodamines, coumarins and dyes.

Moieties or groups that enhance the pharmacodynamic properties include those that improve uptake, enhance resistance to degradation, and/or strengthen sequence-specific hybridization with the target nucleic acid.

Moieties or groups that enhance the pharmacokinetic properties include those that improve uptake, distribution, metabolism or excretion of the antisense compounds.

Representative moieties or groups are disclosed in PCT/US92/09196 and US 6,287,860.

Moieties or groups include but are not limited to lipid moieties such as a cholesterol moiety, cholic acid, a thioether, for example, hexyl-S-tritylthiol, a thiocholesterol, an aliphatic chain, for example, dodecandiol or undecyl residues, a phospholipid, for example, di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety.

Antisense compounds useful in the methods of the present disclosure may also be conjugated to active drug substances. This includes the Somatostatin agonist, for example, Octreotide, Lanreotide or Pasireotide. Techniques to conjugate to Octreotide are known to those skilled in the art.

Oligonucleotide-drug conjugates and their preparation are described in US 09/334,130.

Representative United States patents that teach the preparation of such conjugates include, but are not limited to, US 4,828,979, US 4,948,882, US 5,218,105, US 5,525,465, US 5,541,313, US 5,545,730, US 5,552,538, US 5,578,717, US 5,580,731, US 5,580,731, US 5,591,584, US 5,109,124, US 5,118,802, US 5,138,045, US 5,414,077, US 5,486,603, US 5,512,439, US 5,578,718, US 5,608,046, US 4,587,044, US 4,605,735, US 4,667,025, US 4,762,779, US 4,789,737, US 4,824,941, US 4,835,263, US 4,876,335, US 4,904,582, US 4,958,013, US 5,082,830, US 5,112,963, US 5,214,136, US 5,082,830, US 5,112,963, US 5,214,136, US 5,245,022, US 5,254,469, US 5,258,506, US 5,262,536, US 5,272,250, US 5,292,873, US 5,317,098, US 5,371,241, US 5,391,723, US 5,416,203, US 5,451,463, US 5,510,475, US 5,512,667, US 5,514,785, US 5,565,552, US 5,567,810, US 5,574,142, US 5,585,481, US 5,587,371, US 5,595,726, US 5,597,696, US 5,599,923, US 5,599,928 and US 5,688,941.

### Chimeric compounds

As would be appreciated by those skilled in the art, it is not necessary for all positions in a given compound to be uniformly modified and in fact, more than one of the aforementioned modifications may be incorporated in a single oligonucleotide or even at a single nucleoside within an oligonucleotide.

Antisense compounds useful in the methods of the present disclosure include chimeric oligonucleotides. "Chimeric oligonucleotides" contain two or more chemically distinct regions, each made up of at least one monomer unit, that is, a nucleotide in the case of an oligonucleotide compound. These oligonucleotides typically contain at least one region wherein the oligonucleotide is modified so as to confer upon the oligonucleotide increased resistance to nuclease degradation, increased cellular uptake, increased stability and/or increased binding affinity for the target nucleic acid. An additional region of the oligonucleotide may serve as a substrate for enzymes capable of cleaving RNA:DNA or RNA:RNA hybrids. By way of example, RNAse H is a cellular endonuclease which cleaves the RNA strand of an RNA:DNA duplex. Activation of RNase H, therefore, results in cleavage of the RNA target, thereby greatly enhancing the efficiency of oligonucleotide-mediated inhibition of gene expression. The cleavage of RNA:RNA hybrids can, in like fashion, be accomplished through the actions of endoribonucleases, such as RNAseL which cleaves both cellular and viral RNA. Cleavage of the RNA target can be routinely detected by gel electrophoresis and if necessary, associated nucleic acid hybridization techniques known in the art.

Chimeric antisense compounds useful in the methods of the present disclosure may be formed as composite structures of two or more oligonucleotides, modified oligonucleotides, and/or oligonucleotide mimetics. Such compounds have also been referred to in the art as hybrids or gapmers.

Representative United States patents that teach the preparation of such hybrid structures include, but are not limited to, US 5,013,830, US 5,149,797, US 5,220,007, US 5,256,775, US 5,366,878, US 5,403,711, US 5,491,133, US 5,565,350, US 5,623,065, US 5,652,355, US 5,652,356, and US 5,700,922.

### Exemplary oligonucleotides

In one embodiment of the present disclosure, the antisense compound is a second generation phosphorothioate backbone 2'-MOE-modified chimeric oligonucleotide gapmer designed to hybridize to GHR mRNA.

Exemplary oligonucleotides are shown in Table 1. "Target site" indicates the first (5'-most) nucleotide number on the particular target sequence to which the oligonucleotide binds. "% Inhib" indicates the inhibitory effect on hGHR mRNA levels by quantitative real-time PCR. Data are averages from three experiments in which MCF7 cells were treated with the antisense oligonucleotides.

**Table 1: Inhibition of human growth hormone receptor mRNA levels by chimeric phosphorothioate oligonucleotides having 2'-MOE wings and a deoxy gap**

| **ISIS #** | **REGION** | **TARGET SEQ ID NO** | **TARGET SITE** | **SEQUENCE** | **% INHIB** | **SEQ ID NO** |
|---|---|---|---|---|---|---|
| 227452 | Coding | 4 | 332 | tcagggcattctttccattc | 79 | 6 |
| 227453 | Coding | 4 | 337 | cataatcagggcattctttc | 52 | 7 |
| 227464 | Coding | 4 | 947 | cctttaatctttggaactgg | 58 | 8 |
| 227468 | Coding | 4 | 1079 | tcatcaatatctagctcaat | 62 | 9 |
| 227469 | Coding | 4 | 1124 | cttagaagtctgtctgtgtc | 63 | 10 |
| 227475 | Coding | 4 | 1514 | cctgctggtgtaatgtcgct | 68 | 11 |
| 227480 | Coding | 4 | 1724 | atgtaaatgtcctcttggtt | 66 | 12 |
| 227481 | Coding | 4 | 1729 | tggtgatgtaaatgtcctct | 45 | 13 |
| 227482 | Coding | 4 | 1734 | ttctgtggtgatgtaaatgt | 53 | 14 |
| 227483 | Coding | 4 | 1739 | aggctttctgtggtgatgta | 75 | 15 |
| 227484 | Coding | 4 | 1744 | tggtaaggctttctgtggtg | 63 | 16 |
| 227488 | Coding | 4 | 1922 | agttggtctgtgctcacata | 86 | 17 |
| 227489 | Coding | 4 | 1927 | tgttcagttggtctgtgctc | 75 | 18 |
| 227490 | Coding | 4 | 1936 | gcatgattttgttcagttgg | 67 | 19 |
| 227499 | 3'UTR | 4 | 2656 | tataaaagggctttgtaaaa | 14 | 20 |
| 227500 | 3'UTR | 4 | 4043 | catagcagcaaagtagcaga | 69 | 21 |
| 227501 | 3'UTR | 4 | 4183 | gctatttttggctatagaaa | 64 | 22 |
| 227502 | 3'UTR | 4 | 4197 | gattgaggtatttagctatt | 56 | 23 |
| 272302 | Start Codon | 4 | 31 | gatccatacctgtaggacct | 60 | 24 |
| 272303 | Start Codon | 4 | 36 | ccagagatccatacctgtag | 55 | 25 |
| 272304 | Coding | 4 | 115 | tgctaaggatagctgctgtg | 48 | 26 |
| 272305 | Coding | 4 | 160 | ttgtctttaggcctggatta | 68 | 27 |
| 272306 | Coding | 4 | 170 | ttagaagaatttgtctttag | 13 | 28 |
| 272307 | Coding | 4 | 185 | gtgaatttaggctccttaga | 55 | 29 |
| 272308 | Coding | 4 | 274 | gctgtatgggtcctaggttc | 57 | 30 |
| 272309 | Coding | 4 | 362 | taacagctgttttccccagc | 85 | 31 |
| 272310 | Coding | 4 | 439 | tttcatccactgtaccacca | 76 | 32 |
| 272311 | Coding | 4 | 468 | ttgcactatttcatcaacag | 47 | 33 |
| 272312 | Coding | 4 | 480 | gggtggatctggttgcacta | 57 | 34 |
| 272313 | Coding | 4 | 564 | attgcgtggtgcttcccatc | 77 | 35 |
| 272314 | Coding | 4 | 652 | tagggtccatcattttccat | 56 | 36 |
| 272315 | Coding | 4 | 684 | caatgagtacactggaactg | 53 | 37 |
| 272316 | Coding | 4 | 752 | aactcgccataatttccaga | 64 | 38 |
| 272317 | Coding | 4 | 857 | agcccaaatattccaaagat | 65 | 39 |
| 272318 | Coding | 4 | 913 | tcagcattttaatcctttgc | 55 | 40 |
| 272319 | Coding | 4 | 979 | attttccttccttgaggaga | 67 | 41 |
| 272320 | Coding | 4 | 1000 | agattgtgttcacctcctct | 70 | 42 |
| 272321 | Coding | 4 | 1053 | aacccaagagtcatcactgt | 64 | 43 |
| 272322 | Coding | 4 | 1084 | ctggctcatcaatatctagc | 84 | 44 |
| 272323 | Coding | 4 | 1110 | tgtgtctgattcctcagtct | 67 | 45 |
| 272324 | Coding | 4 | 1236 | tatgtcattggcattgaaat | 53 | 46 |
| 272325 | Coding | 4 | 1302 | aaggcataagagatctgctt | 66 | 47 |
| 272326 | Coding | 4 | 1420 | actcagctccttcagtagga | 77 | 48 |
| 272327 | Coding | 4 | 1560 | ggacatccctgccttattct | 60 | 49 |
| 272328 | Coding | 4 | 1623 | ggcattgtccataaggaagt | 85 | 50 |
| 272329 | Coding | 4 | 1651 | actttttggcatctgcctca | 63 | 51 |
| 272330 | Coding | 4 | 1656 | gatgcactttttggcatctg | 47 | 52 |
| 272331 | Coding | 4 | 1861 | cagtcgcattgagtatgagg | 67 | 53 |
| 272332 | Coding | 4 | 1884 | ctctttgtcaggcaagggca | 75 | 54 |
| 272333 | Coding | 4 | 1913 | gtgctcacatagccacatga | 72 | 55 |
| 272334 | Stop Codon | 4 | 1949 | aagaaaggctaaggcatgat | 61 | 56 |
| 272335 | 3'UTR | 4 | 1973 | aaatacgtagctcttgggaa | 47 | 57 |
| 272336 | 3'UTR | 4 | 2196 | caatcactgctactaaacag | 69 | 58 |
| 272337 | 3'UTR | 4 | 2249 | aaacatagccattcaatgct | 39 | 59 |
| 272338 | 3'UTR | 4 | 2337 | gtgctatggtttgcattcaa | 78 | 60 |
| 272339 | 3'UTR | 4 | 2454 | gttttacatatccaaactat | 72 | 61 |
| 272340 | 3'UTR | 4 | 2853 | catcaaccaagatttggtga | 69 | 62 |
| 272341 | 3'UTR | 4 | 2988 | gaggctatagatcttatctc | 65 | 63 |
| 272342 | 3'UTR | 4 | 3271 | tagtgagaaagaaagtttct | 45 | 64 |
| 272343 | 3'UTR | 4 | 3765 | aatgctctcaagaatgatgt | 48 | 65 |
| 272344 | 3'UTR | 4 | 3980 | acactcaattctagcttttc | 60 | 66 |
| 272345 | 3'UTR | 4 | 4011 | catctattacaaataacatg | 24 | 67 |
| 272346 | 3'UTR | 4 | 4057 | ctcttggagaaaaccatagc | 67 | 68 |
| 272347 | 3'UTR | 4 | 4097 | tctacactgatgatacttta | 62 | 69 |
| 272348 | 3'UTR | 4 | 4120 | cacagctttgaattgaatta | 57 | 70 |
| 272349 | 3'UTR | 4 | 4133 | agtcttccaaacacacagct | 68 | 71 |
| 272350 | 3'UTR | 4 | 4156 | aggctgttgtgaaatagtaa | 67 | 72 |
| 272351 | 3'UTR | 4 | 4170 | atagaaatgttgtcaggctg | 57 | 73 |
| 272352 | 3'UTR | 4 | 4218 | ccaaaatgacattctgagac | 77 | 74 |
| 272353 | 3'UTR | 4 | 4245 | ataatggcttatgtggccac | 72 | 75 |
| 272354 | intron | 5 | 2571 | agttatgtgaccctgattga | 65 | 76 |
| 272355 | intron: exon junction | 5 | 6418 | ttgagtgttcctaaaatgaa | 24 | 77 |
| 272356 | intron | 5 | 8405 | atggaggctggaggttcaaa | 63 | 78 |
| 272357 | intron: exon junction | 5 | 22712 | tagggtccatctttcaagac | 62 | 79 |
| 272358 | intron | 5 | 25543 | tctccagatagaatctaaac | 53 | 80 |
| 272359 | intron | 5 | 29755 | tccaaatattctggtacttt | 72 | 81 |
| 272360 | exon: intron junction | 5 | 29935 | tattagttaccttgaggaga | 0 | 82 |
| 272361 | intron: exon junction | 5 | 30267 | attttccttcctagaaaata | 10 | 83 |

All oligonucelotides in Table 1 are chimeric oligonucleotides ("gapmers"), 20 nucleotides in length, composed of a central "gap" region consisting of ten 2'-deoxynucleotides, which is flanked on both sides (5' and 3' directions) by five-nucleotide "wings". The wings are composed of 2'-methoxyethyl (2'-MOE) nucleotides. The internucleoside (backbone) linkages are phosphorothioate (P=S) throughout the oligonucleotide. All uracils are 5-methyluracils (^{Me}U). Typically, the oligonucleotide is synthesized using 2-methoxyethyl modified thymidines not 5-methyluracils. All pyrimidines are C5 methylated (i.e., U, T, C are C5 methylated).

The oligonucleotide may be synthesized by a multi-step process that may be divided into two distinct operations: solid-phase synthesis and downstream processing. In the first operation, the nucleotide sequence of the oligonucleotide is assembled through a computer-controlled solid-phase synthesizer. Subsequent downstream processing includes deprotection steps, preparative reversed-phase chromatographic purification, isolation and drying to yield the oligonucleotide drug substance. The chemical synthesis of the oligonucelotide utilizes phosphoramidite coupling chemistry followed by oxidative sulfurization and involves sequential coupling of activated monomers to an elongating oligomer, the 3'-terminus of which is covalently attached to the solid support.

### Detritylation (reaction a).

Each cycle of the solid-phase synthesis commences with removal of the acid-labile 5'-O-4, 4'-dimethoxytrityl (DMT) protecting group of the 5' terminal nucleoside of the support bound oligonucleotide. This is accomplished by treatment with an acid solution (e.g., dichloroacetic acid (DCA) in toluene). Following detritylation, excess reagent is removed from the support by washing with acetonitrile in preparation for the next reaction.

### Coupling (reaction b)

Chain elongation is achieved by reaction of the 5'-hydroxyl group of the support-bound oligonucleotide with a solution of the phosphoramidite corresponding to that particular base position (e.g., for base2: MOE-^{Me}C amidite) in the presence of an activator (e.g., 1H-tetrazole). This results in the formation of a phosphite triester linkage between the incoming nucleotide synthon and the support-bound oligonucleotide chain. After the coupling reaction, excess reagent is removed from the support by washing with acetonitrile in preparation for the next reaction.

### Sulfurization (reaction c)

The newly formed phosphite triester linkage is converted to the corresponding [O, O, O)-trialkyl phosphorothioate triester by treatment with a solution of a sulfur transfer reagent (e.g., phenylacetyl disulfide). Following sulfurization, excess reagent is removed from the support by washing with acetonitrile in preparation for the next reaction.

### Capping (reaction d)

A small proportion of the 5'-hydroxy groups available in any given cycle fail to extend. Coupling of these groups in any of the subsequent cycles would result in formation of process-related impurities ("DMT-on (n-1)-mers") which are difficult to separate from the desired product. To prevent formation of these impurities and to facilitate purification, a "capping reagent" (e.g., acetic anhydride and N-methylimidazole/acetonitrile/pyridine) is introduced into the reactor vessel to give capped sequences. The resulting failure sequences ("DMT-off shortmers") are separated from the desired product by reversed phase HPLC purification. After the capping reaction, excess reagent is removed from the support by washing with acetonitrile in preparation of the next reaction.

Reiteration of this basic four-step cycle using the appropriate protected nucleoside phosphoramidite allows assembly of the entire protected oligonucleotide sequence.

### Backbone deprotection (reaction e)

Following completion of the assembly portion of the process the cyanoethyl groups protecting the (O, O, O)-trialkyl phosphorothioate triester internucleotide linkages are removed by treatment with a solution of triethylamine (TEA) in acetonitrile. The reagent and acrylonitrile generated during this step are removed by washing the column with acetonitrile.

### Cleavage from support and base deprotection (reaction f)

Deprotection of the exocyclic amino groups and cleavage of the crude product from the support is achieved by incubation with aqueous ammonium hydroxide (reaction f). Purification of the crude, 5'-O-DMT-protected product is accomplished by reversed phase HPLC. The reversed phase HPLC step removes DMT-off failure sequences. The elution profile is monitored by UV absorption spectroscopy. Fractions containing DMT-on oligonucleotide product are collected and analyzed.

### Acidic deprotection (reaction g)

Reversed phase HPLC fractions containing 5'-O-DMT-protected oligonucleotide are pooled and transferred to a precipitation tank. The products obtained from the purification of several syntheses are combined at this stage of the process. Purified DMT-on oligonucleotide is treated with acid (e.g., acetic acid) to remove the DMT group attached to the 5' terminus. After acid exposure for the prescribed time and neutralization, the oligonucleotide drug substance is isolated and dried.

Following the final acidic deprotection step, the solution is neutralized by addition of aqueous sodium hydroxide and the oligonucleotide drug substance is precipitated from solution by adding ethanol. The precipitated material is allowed to settle at the bottom of the reaction vessel and the ethanolic supernatant decanted. The precipitated material is redissolved in purified water and the solution pH adjusted to between pH 7.2 and 7.3. The precipitation step is repeated. The precipitated material is dissolved in water and the solution filtered through a 0.45 micron filter and transferred into disposable polypropylene trays that are then loaded into a lyophilizer. The solution is cooled to -50°C. Primary drying is carried out at 25°C for 37 hours. The temperature is increased to 300°C and a secondary drying step performed for 5.5 hours. Following completion of the lyophilization process, the drug substance is transferred to high density polyethylene bottles and stored at -200°C.

### Target nucleic acid

"Targeting" an antisense compound to a particular nucleic acid can be a multistep process. The process usually begins with the identification of a target nucleic acid whose function is to be modulated. In the present disclosure, the target nucleic acid encodes growth hormone receptor (GHR). The term "target nucleic acid" encompasses DNA encoding GHR, RNA (including pre-mRNA and mRNA or portions thereof) transcribed from such DNA, and further, cDNA derived from such RNA.

The cDNA encoding the growth hormone receptor has been cloned from many species. The receptor consists of an extracellular hormone-binding region (exons 2-7), a single membrane spanning region (exon 8), and an intracellular region (exons 9-10). There are also multiple alternative 5' untranslated regions which are alternative first exons of the gene, in both the human and mouse transcripts. Growth hormone receptor has no intrinsic kinase domain, but the intracellular region plays a major role in the signal transduction process. A truncated form of the receptor, known as growth hormone binding protein (GHBP), lacks the transmembrane and intracellular regions of GHR and is secreted into the serum. The truncated protein is produced by one of two different processes, depending on the animal species. In mice and rats, alternative splicing of GHR precursor messenger RNA replaces the transmembrane and intracellular regions with a very short hydrophilic tail (encoded by exon 8A). In humans, cows, and pigs (among others), no alternative RNA splicing is apparent but instead the GHBP is produced by proteolysis of the GHR. The GHBP appears to be to modulate the level of circulating growth hormone (GH).

In one embodiment of the present disclosure, the GHR is a human GHR (hGHR) having a nucleotide sequence as shown in NM_000163.4 (SEQ ID NO:4) or NG_011688 (4852-302955) (SEQ ID NO:5).

The targeting process usually also includes determination of at least one target region, segment, or site within the target nucleic acid for the antisense interaction to occur such that the desired effect, for example, inhibition of expression, will result. The term "region" as used herein is defined as a portion of the target nucleic acid having at least one identifiable structure, function, or characteristic. Within regions of the target nucleic acids are segments. "Segments" are defined as smaller or sub-portions of regions within a target nucleic acid. "Sites" as used herein, means positions within the target nucleic acid.

Since the "translation initiation codon" is typically 5'-AUG (in transcribed mRNA molecules; 5'-ATG in the corresponding DNA molecule), the translation initiation codon is also referred to as the "AUG codon", the "start codon" or the "AUG start codon". A minority of genes have a translation initiation codon having the RNA sequence 5'-GUG, 5'-UUG, or 5'-CUG, and 5'-AUA, 5'-ACG and 5'-CUG have been shown to function in vivo. Thus, the terms "translation initiation codon" and "start codon" can encompass many codon sequences even though the initiator amino acid in each instance is typically methionine (in eukaryotes) or formylmethionine (in prokaryotes). It is also known in the art that eukaryotic and prokaryotic genes may have two or more alternative start codons, any one of which may be preferentially utilized for translation initiation in a particular cell type or tissue, or under a particular set of conditions. The terms "start codon" and "translation initiation codon" as used herein refer to the codon or codons that are used in vivo to initiate translation of an mRNA transcribed from a gene encoding, for example, GHR, regardless of the sequence(s) of such codons.

A "translation termination codon" also referred to as a "stop codon" may have one of three RNA sequences: 5'-UAA, 5'-UAG and 5'-UGA (5'-TAA, 5'-TAG and 5'-TGA, respectively in the corresponding DNA molecule). The terms "translation termination codon" and "stop codon" as used herein refer to the codon or codons that are used *in vivo* to terminate translation of an mRNA transcribed from a gene encoding the GHR, regardless of the sequence(s) of such codons.

The terms "start codon region" and "translation initiation codon region" refer to a portion of the mRNA or gene that encompasses from about 25 to about 50 contiguous nucleotides in either direction (i.e., 5' or 3') from the translation initiation codon. Similarly, the terms and "stop codon region" and "translation termination codon region" refer to a portion of the mRNA or gene that encompasses from about 25 to about 50 contiguous nucleotides in either direction (i.e., 5' or 3') from the translation termination codon. Consequently, the "start codon region" or "translation initiation codon region" and the "stop codon region" or "translation termination codon region" are all regions which may be targeted effectively with the antisense compounds.

The "open reading frame" (ORF) or "coding region", which is known in the art to refer to the region between the translation initiation codon and the translation termination codon, is also a region which may be targeted effectively. In one embodiment of the present disclosure, the intragenic region encompassing the translation initiation or termination codon of the ORF of a gene is targeted.

Other target regions include the 5' untranslated region (5'UTR), known in the art to refer to the portion of the mRNA in the 5' direction from the translation initiation codon, and thus including nucleotides between the 5' cap site and the translation initiation codon of the mRNA (or corresponding nucleotides on the gene), and the 3' untranslated region (3'UTR), known in the art to refer to the portion of the mRNA in the 3' direction from the translation termination codon, and thus including nucleotides between the translation termination codon and 3' end of the mRNA (or corresponding nucleotides on the gene). The 5' cap site of an mRNA comprises an N7-methylated guanosine residue joined to the 5'-most residue of the mRNA via a 5'-5' triphosphate linkage. The 5' cap region of an mRNA is considered to include the 5' cap structure itself, as well as the first 50 nucleotides adjacent to the cap site. In one embodiment of the present disclosure, the 5' cap region is targeted.

Although some eukaryotic mRNA transcripts are directly translated, many contain one or more regions, known as "introns," which are excised from a transcript before it is translated. The remaining (and therefore translated) regions are known as "exons" and are spliced together to form a continuous mRNA sequence. mRNA transcripts produced via the process of splicing of two (or more) mRNAs from different gene sources are known as "fusion transcripts". In one embodiment of the present disclosure, introns, or splice sites, that is, intron-exon junctions or exon-intron junctions, or aberrant fusion junctions due to rearrangements or deletions are targeted.

Alternative RNA transcripts can be produced from the same genomic region of DNA. These alternative transcripts are generally known as "variants".

"Pre-mRNA variants" are transcripts produced from the same genomic DNA that differ from other transcripts produced from the same genomic DNA in either their start or stop position and contain both intronic and exonic sequence. Upon excision of one or more exon or intron regions, or portions thereof during splicing, pre-mRNA variants produce smaller "mRNA variants". Consequently, mRNA variants are processed pre-mRNA variants and each unique pre-mRNA variant must always produce a unique mRNA variant as a result of splicing. These mRNA variants are also known as "alternative splice variants". If no splicing of the pre-mRNA variant occurs then the pre-mRNA variant is identical to the mRNA variant.

In mouse, rat and monkey, GHBP, which is the soluble shortened form of GHR, is produced by alternative splicing of the GHR primary transcript. In some embodiments of the present disclosure, it may be preferable to target regions of the transcript which are present in both the GHR transcript and in the shorter GHBP transcript. In other embodiments of the present disclosure it may be preferable to target regions of the mRNA which are only present in the longer GHR transcript. In humans, cows, and pigs (among others), no alternative RNA splicing is apparent but instead the shorter GHBP is produced by proteolysis of the GHR. It will be understood that in the context of this disclosure, "nucleic acid encoding GHR" includes nucleic acid encoding GHBP.

Variants can be produced through the use of alternative signals to start or stop transcription, that is, through use of an alternative start codon or stop codon. Variants that originate from a pre-mRNA or mRNA that use alternative start codons are known as "alternative start variants" of that pre-mRNA or mRNA. Those transcripts that use an alternative stop codon are known as "alternative stop variants" of that pre-mRNA or mRNA. One specific type of alternative stop variant is the "polyA variant" in which the multiple transcripts produced result from the alternative selection of one of the "polyA stop signals" by the transcription machinery, thereby producing transcripts that terminate at unique polyA sites. In one embodiment of the present disclosure, the pre-mRNA or mRNA variants are targeted. The human GHR has several transcript variants as can be identified from the National Center for Biotechnology Information http://www.ncbi.nlm.nih.gov/guide/ and other web sites http://www.uniprot.org/uniprot/P10912#PRO_0000010958. There are additionally alternative sequences and natural variants sequences of these transcripts.

The location on the target nucleic acid to which the antisense compound hybridizes is referred to as the "target segment". As used herein, the term "target segment" is defined as at least an 8-nucleobase portion of a target region to which an antisense compound is targeted. While not wishing to be bound by theory, it is presently believed that these target segments represent portions of the target nucleic acid which are accessible for hybridization.

Once one or more target regions, segments or sites have been identified, antisense compounds are chosen which are sufficiently complementary to a target segment, that is, antisense compounds that hybridize sufficiently well and with sufficient specificity, to give the desired effect.

In a further embodiment of the present disclosure, the target segment identified herein may be employed in a screen for additional compounds that modulate the expression of the GHR gene (and thus expression of GHR). "Modulators" are those compounds that decrease or increase the expression of a nucleic acid molecule encoding GHR and which comprise at least a 8 nucleobase portion which is complementary to a preferred target segment.

The screening method comprises the steps of contacting a target segment of the nucleic acid encoding GHR with one or more candidate modulators, and selecting for one or more candidate modulators which decrease or increase the expression of a nucleic acid encoding GHR. Once it is shown that the candidate modulator or modulators are capable of modulating (e.g., either decreasing or increasing) the expression of a nucleic acid encoding GHR, the modulator may then be employed in further investigative studies of the function of GHR, or for use as a research, diagnostic, or therapeutic agent.

The target segment may also be combined with its respective complementary antisense compound to form stabilized double-stranded (duplexed) oligonucleotides.

Such double stranded oligonucleotide moieties have been shown in the art to modulate target expression and regulate translation, as well as RNA processing via an antisense mechanism. Moreover, the double-stranded moieties may be subject to chemical modifications (Fire et al., 1998; Timmons and Fire, 1998; Timmons et al., 2001; Tabara et al., 1998; Montgomery et al., 1998; Tuschl et al., 1999; Elbashir et al., 2001a; Elbashir et al., 2001b). For example, such double-stranded moieties have been shown to inhibit the target by the classical hybridization of antisense strand of the duplex to the target, thereby triggering enzymatic degradation of the target (Tijsterman et al., 2002).

### Exemplary target nucleic acids

Exemplary target sequences are shown in Table 2.

**Table 2: Sequence and position of preferred target segments identified in growth hormone receptor**

| **SITE ID** | **TARGET SEQ ID NO** | **TARGET SITE** | **SEQUENCE** | **REV COMP OF SEQ ID** | **ACTIVE IN** | **SEQ ID NO** |
|---|---|---|---|---|---|---|
| 144070 | 4 | 332 | gaatggaaagaatgccctga | 19 | *H. sapiens* | 84 |
| 144071 | 4 | 337 | gaaagaatgccctgattatg | 20 | *H. sapiens* | 85 |
| 144082 | 4 | 947 | ccagttccaaagattaaagg | 21 | *H. sapiens* | 86 |
| 144086 | 4 | 1079 | attgagctagatattgatga | 22 | *H. sapiens* | 87 |
| 144087 | 4 | 1124 | gacacagacagacttctaag | 23 | *H. sapiens* | 88 |
| 144093 | 4 | 1514 | agcgacattacaccagcagg | 24 | *H. sapiens* | 89 |
| 144098 | 4 | 1724 | aaccaagaggacatttacat | 25 | *H. sapiens* | 90 |
| 144099 | 4 | 1729 | agaggacatttacatcacca | 26 | *H. sapiens* | 91 |
| 144100 | 4 | 1734 | acatttacatcaccacagaa | 27 | *H. sapiens* | 92 |
| 144101 | 4 | 1739 | tacatcaccacagaaagcct | 28 | *H. sapiens* | 93 |
| 144102 | 4 | 1744 | caccacagaaagccttacca | 29 | *H. sapiens* | 94 |
| 144106 | 4 | 1922 | tatgtgagcacagaccaact | 30 | *H. sapiens* | 95 |
| 144107 | 4 | 1927 | gagcacagaccaactgaaca | 31 | *H. sapiens* | 96 |
| 144108 | 4 | 1936 | ccaactgaacaaaatcatgc | 32 | *H. sapiens* | 97 |
| 144118 | 4 | 4043 | tctgctactttgctgctatg | 34 | *H. sapiens* | 98 |
| 144119 | 4 | 4183 | tttctatagccaaaaatagc | 35 | *H. sapiens* | 99 |
| 144120 | 4 | 4197 | aatagctaaatacctcaatc | 36 | *H. sapiens* | 100 |
| 188518 | 4 | 31 | aggtcctacaggtatggatc | 37 | *H. sapiens* | 101 |
| 188519 | 4 | 36 | ctacaggtatggatctctgg | 38 | *H. sapiens* | 102 |
| 188520 | 4 | 115 | cacagcagctatccttagca | 39 | *H. sapiens* | 103 |
| 188521 | 4 | 160 | taatccaggcctaaagacaa | 40 | *H. sapiens* | 104 |
| 188523 | 4 | 185 | tctaaggagcctaaattcac | 42 | *H. sapiens* | 105 |
| 188524 | 4 | 274 | gaacctaggacccatacagc | 43 | *H. sapiens* | 106 |
| 188525 | 4 | 362 | gctggggaaaacagctgtta | 44 | *H. sapiens* | 107 |
| 188526 | 4 | 439 | tggtggtacagtggatgaaa | 45 | *H. sapiens* | 108 |
| 188527 | 4 | 468 | ctgttgatgaaatagtgcaa | 46 | *H. sapiens* | 109 |
| 188528 | 4 | 480 | tagtgcaaccagatccaccc | 47 | *H. sapiens* | 110 |
| 188529 | 4 | 564 | gatgggaagcaccacgcaat | 48 | *H. sapiens* | 111 |
| 188530 | 4 | 652 | atggaaaatgatggacccta | 49 | *H. sapiens* | 112 |
| 188531 | 4 | 684 | cagttccagtgtactcattg | 50 | *H. sapiens* | 113 |
| 188532 | 4 | 752 | tctggaaattatggcgagtt | 51 | *H. sapiens* | 114 |
| 188533 | 4 | 857 | atctttggaatatttgggct | 52 | *H. sapiens* | 115 |
| 188534 | 4 | 913 | gcaaaggattaaaatgctga | 53 | *H. sapiens* | 116 |
| 188535 | 4 | 979 | tctcctcaaggaaggaaaat | 54 | *H. sapiens* | 117 |
| 188536 | 4 | 1000 | agaggaggtgaacacaatct | 55 | *H. sapiens* | 118 |
| 188537 | 4 | 1053 | acagtgatgactcttgggtt | 56 | *H. sapiens* | 119 |
| 188538 | 4 | 1084 | gctagatattgatgagccag | 57 | *H. sapiens* | 120 |
| 188539 | 4 | 1110 | agactgaggaatcagacaca | 58 | *H. sapiens* | 121 |
| 188540 | 4 | 1236 | atttcaatgccaatgacata | 59 | *H. sapiens* | 122 |
| 188541 | 4 | 1302 | aagcagatctcttatgcctt | 60 | *H. sapiens* | 123 |
| 188542 | 4 | 1420 | tcctactgaaggagctgagt | 61 | *H. sapiens* | 124 |
| 188543 | 4 | 1560 | agaataaggcagggatgtcc | 62 | *H. sapiens* | 125 |
| 188544 | 4 | 1623 | acttccttatggacaatgcc | 63 | *H. sapiens* | 126 |
| 188545 | 4 | 1651 | tgaggcagatgccaaaaagt | 64 | *H. sapiens* | 127 |
| 188546 | 4 | 1656 | cagatgccaaaaagtgcatc | 65 | *H. sapiens* | 128 |
| 188547 | 4 | 1861 | cctcatactcaatgcgactg | 66 | *H. sapiens* | 129 |
| 188548 | 4 | 1884 | tgcccttgcctgacaaagag | 67 | *H. sapiens* | 130 |
| 188549 | 4 | 1913 | tcatgtggctatgtgagcac | 68 | *H. sapiens* | 131 |
| 188550 | 4 | 1949 | atcatgccttagcctttctt | 69 | *H. sapiens* | 132 |
| 188551 | 4 | 1973 | ttcccaagagctacgtattt | 70 | *H. sapiens* | 133 |
| 188552 | 4 | 2196 | ctgtttagtagcagtgattg | 71 | *H. sapiens* | 134 |
| 188554 | 4 | 2337 | ttgaatgcaaaccatagcac | 73 | *H. sapiens* | 135 |
| 188555 | 4 | 2454 | atagtttggatatgtaaaac | 74 | *H. sapiens* | 136 |
| 188556 | 4 | 2853 | tcaccaaatcttggttgatg | 75 | *H. sapiens* | 137 |
| 188557 | 4 | 2988 | gagataagatctatagcctc | 76 | *H. sapiens* | 138 |
| 188558 | 4 | 3271 | agaaactttctttctcacta | 77 | *H. sapiens* | 139 |
| 188559 | 4 | 3765 | acatcattcttgagagcatt | 78 | *H. sapiens* | 140 |
| 188560 | 4 | 3980 | gaaaagctagaattgagtgt | 79 | *H. sapiens* | 141 |
| 188562 | 4 | 4057 | gctatggttttctccaagag | 81 | *H. sapiens* | 142 |
| 188563 | 4 | 4097 | taaagtatcatcagtgtaga | 82 | *H. sapiens* | 143 |
| 188564 | 4 | 4120 | taattcaattcaaagctgtg | 83 | *H. sapiens* | 144 |
| 188565 | 4 | 4133 | agctgtgtgtttggaagact | 84 | *H. sapiens* | 145 |
| 188566 | 4 | 4156 | ttactatttcacaacagcct | 85 | *H. sapiens* | 146 |
| 188567 | 4 | 4170 | cagcctgacaacatttctat | 86 | *H. sapiens* | 147 |
| 188568 | 4 | 4218 | gtctcagaatgtcattttgg | 87 | *H. sapiens* | 148 |
| 188569 | 4 | 4245 | gtggccacataagccattat | 88 | *H. sapiens* | 149 |
| 188570 | 5 | 2571 | tcaatcagggtcacataact | 89 | *H. sapiens* | 150 |
| 188572 | 5 | 8405 | tttgaacctccagcctccat | 91 | *H. sapiens* | 151 |
| 188573 | 5 | 22712 | gtcttgaaagatggacccta | 92 | *H. sapiens* | 152 |
| 188574 | 5 | 25543 | gtttagattctatctggaga | 93 | *H. sapiens* | 153 |
| 188575 | 5 | 29755 | aaagtaccagaatatttgga | 94 | *H. sapiens* | 154 |

### Compositions/Formulations

Antisense compounds useful in the methods of the present disclosure may be admixed, encapsulated, conjugated or otherwise associated with other molecules, molecule structures or mixtures of compounds, resulting in, for example, liposomes, receptor-targeted molecules, oral, rectal, topical or other formulations, for assisting in uptake, distribution and/or absorption.

Representative United States patents that teach the preparation of such uptake, distribution and/or absorption-assisting formulations include, but are not limited to, US 5,108,921, US 5,354,844, US 5,416,016, US 5,459,127, US 5,521,291, US 5,543,158, US 5,547,932, US 5,583,020, US 5,591,721, US 4,426,330, US 4,534,899, US 5,013,556, US 5,108,921, US 5,213,804, US 5,227,170, US 5,264,221, US 5,356,633, US 5,395,619, US 5,416,016, US 5,417,978, US 5,462,854, US 5,469,854, US 5,512,295, US 5,527,528, US 5,534,259, US 5,543,152, US 5,556,948, US 5,580,575, and US 5,595,756.

The antisense compounds may be administered in a pharmaceutically acceptable carrier. The term "pharmaceutically acceptable carrier" refers to molecular entities that do not produce an allergic, toxic or otherwise adverse reaction when administered to a subject, particularly a mammal, and more particularly a human. The pharmaceutically acceptable carrier may be solid or liquid. Useful examples of pharmaceutically acceptable carriers include, but are not limited to, diluents, solvents, surfactants, excipients, suspending agents, buffering agents, lubricating agents, adjuvants, vehicles, emulsifiers, absorbants, dispersion media, coatings, stabilizers, protective colloids, adhesives, thickeners, thixotropic agents, penetration agents, sequestering agents, isotonic and absorption delaying agents that do not affect the activity of the active agents of the disclosure.

The antisense compounds may be pharmaceutically acceptable salts, esters, or salts of the esters, or any other compounds which, upon administration are capable of providing (directly or indirectly) the biologically active metabolite.

The term "pharmaceutically acceptable salts" as used herein refers to physiologically and pharmaceutically acceptable salts of the antisense compounds that retain the desired biological activities of the parent compounds and do not impart undesired toxicological effects upon administration. Preferred examples of pharmaceutically acceptable salts and their uses are further described in US 6,287,860.

The antisense compounds may be prodrugs or pharmaceutically acceptable salts of the prodrugs, or other bioequivalents.

The term "prodrugs" as used herein refers to therapeutic agents that are prepared in an inactive form that is converted to an active form (i.e., drug) upon administration by the action of endogenous enzymes or other chemicals and/or conditions. In particular, prodrug forms of the antisense compounds are prepared as SATE [(S acetyl-2-thioethyl) phosphate] derivatives according to the methods disclosed in WO 93/24510, WO 94/26764 and US 5,770,713.

Somatostatin agonsits may be administered in a pharmaceutically acceptable carrier. The carrier must be "acceptable" in the sense of being compatible with the active ingredient(s) of the formulation (e.g., capable of stabilizing peptides) and not deleterious to the subject to be treated. Desirably, the formulation should not include oxidizing agents or other substances with which peptides are known to be incompatible. For example, somatostatin agonists in the cyclized form (e.g., internal cysteine disulfide bond) are oxidized; thus, the presence of reducing agents as excipients could lead to an opening of the cysteine disulfide bridge. On the other hand, highly oxidative conditions can lead to the formation of cysteine sulfoxide and to the oxidation of tryptophane. Consequently, it is important to carefully select the excipient. pH is another key factor, and it may be necessary to buffer the product under slightly acidic conditions (pH 5 to 6).

The formulations may conveniently be presented in unit dosage form and may be prepared by any of the methods well known in the art of pharmacy. All methods include the step of bringing the active ingredient(s) into association with the carrier which constitutes one or more accessory ingredients.

In general, the formulations for tablets or powders are prepared by uniformly and intimately blending the active ingredient with finely divided solid carriers, and then, if necessary, as in the case of tablets, forming the product into the desired shape and size.

Formulations suitable for parenteral (e.g., intravenous) administration, on the other hand, conveniently comprise sterile aqueous solutions of the active ingredient(s). Preferably, the solutions are isotonic with the blood of the subject to be treated. Such formulations may be conveniently prepared by dissolving active ingredient(s) in a solvent comprising water to produce an aqueous solution, and rendering said solution sterile. The formulation may be presented in unit or multi-dose containers, for example, sealed ampoules or vials.

Formulations suitable for sustained release parenteral administrations (e.g., biodegradable polymer formulations) are also well known in the art (see, for example, U.S. Pat. Nos. 3,773,919 and 4,767,628 and PCT Publication No. WO 94/15587).

Compositions for rectal or vaginal administration are preferably suppositories which may contain, in addition to the active substance, excipients such as coca butter or a suppository wax.

Compositions for nasal or sublingual administration are also prepared with standard excipients well known in the art.

For topical administration, they are best used in the form of solutions, creams, salves, lotions, ointments and the like.

### Administration

The methods of the present disclosure rely on the unsuspected additional activity and/or synergy of combining a Somatostatin analog having Somatostatin receptor agonistic activity with an oligonucleotide targeted to growth hormone receptor (GHR) to reduce insulin-like growth factor I (IGF-I) levels in a subject.

In a particular embodiment of the present disclosure, the Somatostatin agonist the oligonucleotide are administered concomitantly. The Somatostatin agonist and the oligonucleotide may be administered in the form of a composition comprising an admixture of both components. Alternatively, the Somatostatin agonist and the oligonucleotide may be administered in separate compositions.

In one embodiment of the present disclosure, the antisense oligonucelotide is administered systemically. As used herein "systemic administration" is a route of administration that is either enteral or parenteral.

As used herein "enteral" refers to any form of administration that involves any part of the gastrointestinal tract and includes oral administration of, for example, the antisense oligonucleotide in tablet, capsule or drop form; gastric feeding tube, duodenal feeding tube, or gastrostomy; and rectal administration of, for example, the antisense compound in suppository or enema form.

As used herein "parenteral" includes administration by injection or infusion. Examples include, intravenous (into a vein), intraarterial (into an artery), intramuscular (into a muscle), intracardiac (into the heart), subcutaneous (under the skin), intraosseous infusion (into the bone marrow), intradermal, (into the skin itself), intrathecal (into the spinal canal), intraperitoneal (infusion or injection into the peritoneum), intravesical (infusion into the urinary bladder), transdermal (diffusion through the intact skin), transmucosal (diffusion through a mucous membrane), inhalational.

The antisense oligonucleotide may be administered as single dose or as repeated doses on a periodic basis, for example, daily, once every two days, three, four, five, six seven, eight, nine, ten, eleven, twelve, thirteen or fourteen days, once weekly, twice weekly, three times weekly, or every two weeks, every three weeks, or every four weeks.

The antisense oligonucleotide to be used in the therapy is formulated and dosed in a fashion consistent with good medical practice, taking into account the specific condition being treated, the clinical condition of the individual patient, the site of delivery of the oligonucleotide, the method of administration, the scheduling of administration, and other factors known to practitioners. The "effective amount" of oligonucelotide for purposes herein is thus determined by such considerations. The term "effective amount" in this context refers to any dose of the antisense oligonucleotide sufficient to inhibit GHR expression, under the conditions of administration.

By way of example, a dose of 25-3400 mg, more preferably 50-1600 mg oligonucelotide may be administered to a subject over a week. A single dose of 150-400 mg, for example, a dose of 250 mg is particularly contemplated for humans. In one embodiment of the present disclosure, a dose of 250 mg per day is administered six times over 3 weeks, on days 1, 3, 5, 7, 14 and 21 or twice weekly. In another embodiment of the present disclosure, a dose of 250 mg is administered once weekly, or once a fortnight.

Somatostatin agonists may be injected parenterally, for example, intravenously, into the bloodstream of the subject being treated. However, it will be readily appreciated by those skilled in the art that the route, such as subcutaneous, intramuscular, intraperitoneal, enterally, transdermally, transmucously, sustained released polymer compositions (e.g., a lactic acid polymer or lactic-glycolic acid copolymer microparticle or implant), profusion, nasal, oral, topical, vaginal, rectal, nasal, sublingual, etc., will vary with the condition being treated and the activity and bioavailability of the somatostatin agonist being used.

While it is possible for the Somatostatin agonist to be administered as the pure or substantially pure compound, it may also be presented as a pharmaceutical formulation or preparation. The formulations to be used in the present disclosure, for both humans and animals, comprise any of the somatostatin agonists described herein, together with one or more pharmaceutically acceptable carriers thereof, and optionally other therapeutic ingredients.

The Somatostatin agonist may be administered by, for example, continuous infusion (using, for example, minipumps such as osmotic pumps), or by injection using, for example, intravenous or subcutaneous means. In one embodiment of the present disclosure, the Somatostatin agonist is administered subcutaneously. The administration can also be as a single bolus or by slow-release depot formulation. The administration may also be by the oral route, for example using Octreolin™ (oral octreotide acetate).

The Somatostatin agonist to be used in the therapy is formulated and dosed in a fashion consistent with good medical practice, taking into account the specific condition being treated, the clinical condition of the individual patient, the site of delivery of the Somatostatin agonist composition, the method of administration, the scheduling of administration, and other factors known to practitioners. The "effective amount" of Somatostatin agonist for purposes herein is thus determined by such considerations. The term "effective amount" in this context refers to any dose of the Somatostatin analog sufficient to mediate Somatostatin receptor agonistic activity, under the conditions of administration.

The dosage of active ingredient administered in a method of this disclosure may be varied; however, it is necessary that the amount of the active ingredient be such that a suitable dosage form is obtained. The selected dosage depends upon the desired therapeutic effect, on the route of administration, and on the duration of the treatment. Generally, dosage levels of between 0.000001 to 100 mg/kg of body weight daily are administered to humans and other animals, for example, mammals.

A preferred dosage range is 0.01 to 5.0 mg/kg of body weight daily which can be administered as a single dose or divided into multiple doses.

The Somatostatin agonist may be administered as single dose or as repeated doses on a periodic basis, for example, several times a day, daily, once every two days, three, four, five, six seven, eight, nine, ten, eleven, twelve, thirteen or fourteen days, once weekly, twice weekly, three times weekly, or every two weeks, every three weeks, or every four weeks.

In one embodiment of the present disclosure, the Somatostatin agonist is selected from any one of LAR forms, for example, Sandostatin-LAR, Lanreotide Autogel, Pasireotide-LAR, or lanreotide microparticles and the oligonucleotide is ATL1103 (SEQ ID NO:6) and the compounds are administered sequentially. In one embodiment of the present disclosure, the ATL1103 oligonucleotide is first administered at a dose of 250 mg/day on days 1, 3, 5, 7, 14 and 21, and once weekly thereafter (for 5 to 12 weeks) and the Somatostatin agonist is subsequently administered on the same days at the dose of 20 mg/28days for Sandostatin-LAR, 90mg/28days Lanreotide Autogel, 40mg/28days for Pasireotide-LAR, and 20mg/week for Lanreotide microparticles or the higher dose of 30mg/28days for Sandostatin-LAR, or 120mg/28days for Lanreotide Autogel, or 60mg/day for Pasireotide-LAR or 60mg/3weeks for Lanreotide microparticles.

After the 5 to 12 weeks, treatment may be continued (cycle 2) with the same or increased or lower doses of the Somatostatain agonist above, and with the same or increased or lower doses of ATL1103, and with the same or increased or lower dosing frequency to achieve the desired target IGF-I levels. Post 5 to 12 weeks, the Somatostatin agonist dose may be increased from the low dose to the high dose and monitored at about 1 to 4 weeks to assess IGF-I levels. Post 5 to 12 weeks, the ATL1103 dose modification may be in about 25, 50 mg, or 100mg increments and monitored at about 1 to 8 weeks to assess IGF-I levels. Cycle 2 can be continued if the target IGF-I normalization is achieved or a new cycle started to further optimize dosing to achieve IGF-I normalization on a patient by patient basis.

Alternatively, the ATL1103 oligonucleotide may be administered once or twice weekly (for 8 to 12 weeks) at doses of 150, 200, 250, 300, or 350 mg together with the above doses of the Somatostatin agonists. After the 8 to 12 weeks, treatment may be continued (cycle 2) with the same or increased or lower doses of the Somatostatain agonist above, and with the same or increased or lower doses of ATL1103, and with the same or increased or lower dosing frequency to achieve the desired target IGF-I levels. Cycle 2 can be continued if the target IGF-I normalization is achieved or a new cycle started to further optimize dosing to achieve IGF-I normalization on a patient by patient basis.

In another embodiment of the present disclosure, a repeat 21 day treatment cycle may be used, for cancer or retinopathy treatment. The ATL1103 oligonucleotide is first administered at a dose of 250 mg/day on days 1, 3, 5, 7, 14 and 21, and the Somatostatin agonist above is subsequently administered on the same days at one of the doses above. Alternatively, in a repeat cycle, the ATL1103 oligonucleotide dosing may be once or twice weekly 250, 300, or 350 mg together with one of the Somatostatin agonist doses above starting on the same day. Alternatively, treatments may be on different days.

After 5 to 12 weeks, treatment may be continued (cycle 2) with the same or increased or lower doses of Somatostatin agonist, and with the same or increased or lower doses of ATL1103, and administered the same or increased or lower dosing frequency to achieve the desired target IGF-I levels and treatment outcomes in cancer or retinopathy. Post 5 to 12 weeks, the Somatostatin agonist dose modification may be increased from the low dose to the high dose and monitored at about 1 to 4 weeks to assess IGF-I levels. Post 5 to 12 weeks, the ATL1103 dose modification may be in about 25, 50, or 100 mg increments and monitored at about 1 to 8 weeks to assess IGF-I levels. Cycle 2 can be continued if the target IGF-I normalization is achieved or a new cycle started to further optimize dosing to achieve IGF-I normalization on a patient by patient basis.

In another embodiment of the present disclosure, the ATL1103 drug may be dosed once, twice, three times weekly, every other day, or daily at doses of 100, 200, 250, 300, 350, or 400 mg/day and the Somatostatin Octreotide Acetate may be dosed daily, twice daily, or three times daily at the recommended mg/day, or the Somatostatin Octreolin™ may be dosed at the recommended dose 40, 60, or 80mg/day, or the Lanreotide microparticles, Octreotide-LAR, Lanreotide-LAR, Pasireotide LAR administered as recommended.

In another embodiment of the present disclosure, the ATL1103 oligonucleotide is first administered at one of the above doses once every other week and the Somatostatin Lanreotide microparticles is subsequently administered on alternative weeks, so that the patient is on a once weekly alternative dosing regimen, first of ATL1103 and then of Lanreotide microparticles. In a similar embodiment of the present disclosure, the Lanreotide microparticles and ATL1103 may also be combined in a mixture and given on the same day. For example, the ATL1103 in a solution in a pre-filled syringe, may be added to the Lanreotide microparticles, and the mixture administered to the subject.

Somatostatin agonists such as Octreolin, Lanreotide, Pasireotide and Octreotide are administered as short, medium or long acting forms to achieve effective dosages as for example outlined above.

In one embodiment of the present disclosure, ATL1103 (Seq ID 6) is administered in a dose ranging from 4mg/kg/week to 12mg/kg/week. In one embodiment of the present disclosure from 4mg/kg/week to 8mg/kg/week, or 5mg/kg/week to 10mg/kg/week. In some embodiments of the present disclosure, 3.5mg/kg/week, 4mg/kg/week, 4.5mg/kg/week, 5mg/kg/week, 5.5mg/kg/week 6mg/kg/week, 6.5mg/kg/week, 7mg/kg/week, 7.5mg/kg/week, 8mg/kg/week, 8.5mg/kg/week, 9mg/kg/week, 9.5mg/kg/week, 10mg/kg/week 10.5mg/kg/week, 11mg/kg/week, 11.5mg/kg/week, 12mg/kg/week doses are contemplated.

As described in Example 11, ATL1103 is effective in reducing sIGF-I at twice weekly 200mg dosing and is well tolerated. There was a mean reduction of 30% in sIGF-I compared to baseline in the 4 patients with acromegaly treated at twice weekly 200mg, with a mean reduction of 38% in the 3 patients treated that had a body weight below 85kg. As the drug is well tolerated, dosing to beyond twice weekly 200mg to twice weekly 250, twice weekly 300mg, or three times weekly at 200mg, 250mg, and 300mg is proposed. In some embodiments of the present disclosure, once weekly 200, 250, 300, 350, and every other day 200mg or even every day 100mg, or 150mg is employed.

To treat acromegaly patients who have failed to have their sIGF-I normalized with first line Somatostatin therapy it is proposed herein to employ combinations with once weekly doses of 200mg, 250mg, 300mg and 350mg ATL1103 as well as all the hereinabove described doses and dosage intervals together with Somatostatin agonist treatments. Acromegaly patients treated with Somatostatin agonist therapy to normalize their sIGF-I to the upper limit of normal, may also be treated in combinations with ATL1103 to obtain further benefits.

Unit dosage forms of oligonucleotide comprising 50mg, 100mg, 200mg, 250mg, 300mg, 350mg, and 400mg oligonucleotide are contemplated. Unit dosage forms of ATL1103, comprising 200mg, 250mg, 300mg, 350mg, are particularly contemplated.

### EXAMPLES

Examples not falling under the scope of the appended claims do not form part of the invention.

### Example 1: Phase I trial of the GHR targeting drug ATL1103.

The primary objective of the Phase I trial was to assess the safety, tolerability and pharmacokinetics (pK) of ATL1103.

The Phase I trial was a randomized, placebo controlled, double blind study of single ascending doses and multiple doses of ATL1103 in healthy adult male subjects aged between 18 and 45 years. In the single ascending dose stage of the trial, 24 subjects were administered four dose levels of ATL1103 as a single injection starting at 25mg and escalating to 75, 250 and 400mg or placebo. The multiple dose stage was undertaken in 12 subjects, 8 who were to receive six subcutaneous doses of 250mg of ATL1103 and 4 subjects who received placebo administered on days 1, 3, 5, 7, 14 and 21. Subjects were monitored out to day 35.

Importantly, no serious adverse events were reported in this trial. Two subjects in the multiple dose arm withdrew from the study for reasons not related to safety. All adverse events were reported as "mild to moderate". Injection site reactions represented the majority of all the adverse events reported in the trial. There was one elevation in the liver enzyme ALT reported as an adverse event in the multiple dose stage. Importantly, the ALT levels in this subject returned to normal during the dosing phase, suggesting no residual or cumulative effect of the drug on this safety parameter.

A secondary objective of this study was to obtain data on the pharmacodynamic effects of ATL1103 on the IGF-I levels in the blood of the trial subjects. Reduction of increased levels of serum IGF-I to normal is the therapeutic endpoint in the treatment of the growth disorder acromegaly, and reducing the effects of IGF-I has a potential role in the treatment of diabetic retinopathy, nephropathy and certain forms of cancer.

As defined in the statistical analysis plan, the effect of ATL1103 on serum IGF-I was assessed as a change in IGF-I levels versus baseline (starting point) readings for those subjects who received treatment (ATL1103). Pre-dose baseline levels of IGF-I were recorded prior to the commencement of dosing and then measured at weekly intervals until the end of the monitoring period. This treated group showed a trend in reduction in IGF-I levels from day 14 to day 28, with a significant effect (p=.034 one sided t-test) at day 21 with a 7% reduction in mean IGF-I levels versus baseline.

Other exploratory objectives of the study investigated the drug's mechanism of action and broader pharmacological profile, including the pharmacodynamic effects on levels of growth hormone binding protein (GHBP), insulin-like growth factor binding protein 3 (IGFBP-3), acid labile subunit of the insulin-like growth factor binding protein complex (ALS), and growth hormone (GH), as well as *in vitro* mitogenic and apoptotic parameters.

Notably, ATL1103 had a significant effect on reducing GHBP by 16% (p=0.007) at day 21 and 19% (p<0.05) at day 28, one week past the last dose. As circulating GHBP is produced by cleavage from the GHR, the reduction of circulating GHBP levels suggests that GHR expression is being reduced. ATL1103 also significantly reduced IGFBP-3 and ALS, both consistent with its effect on IGF-I and the fact that they are regulated by GH. There was no effect on GH levels. Specific trial details and outcomes are summarized in Tables 3-6.

**Table 3: Summary of ATL1103 Phase I clinical trial**

| | |
|---|---|
| Title | A randomised, placebo-controlled, double-blind, single ascending dose and multiple dose study to assess the safety, tolerability, pharmacokinetics and pharmacodynamics of subcutaneous doses of ATL1103 in healthy adult male subjects |
| Trial description | Phase I trial of subcutaneous administration of ATL1103 in healthy males |
| Objectives | Primary objectives: |
| | To assess the safety and tolerability of single subcutaneous doses (Stage A) and multiple subcutaneous doses (Stage B) of ATL1103 in healthy male subjects. |
| | To determine the single dose and multiple dose pharmacokinetic (PK) profiles of ATL1103 by the subcutaneous route of dosing |
| | Secondary objective: |
| | To assess the pharmacodynamic (PD) effects on IGF-I levels following subcutaneous administration of ATL1103 |
| | Exploratory objective: |
| | To assess the PD effects on circulating levels of (i) growth hormone (GH), (ii) insulin-like growth factor binding protein 3 (IGF-BP3), (iii) insulin-like growth factor acid-labile subunit (ALS), (iv) insulin-like growth factor II (IGF-II) and (v) growth hormone binding protein (GHBP) and on (vi) mitogenic and apoptotic activity, following subcutaneous, administration of ATL1103 |
| Main selection criteria; number of subjects | Males 18 - 45 years of age, BMI: 19 to 30 kg/m2, healthy (determined by medical and drug history, physical examination and ECG). IGF-I levels in the normal range |
| | Stage A: 24 subjects (four groups of six subjects): randomized for 4 on ATL1103 , 2 placebo Stage B: 12 subjects (one group): randomized for 8 active, 4 placebo |
| Test Drug, Dose and Mode of administration | Subcutaneous administration of ATL1103 in the following doses: |
| | Stage A: (single dose): 0 mg (placebo); 25, 75, 250, 400 mg. |
| | Stage B: (multiple doses): 0 mg (placebo); 250 mg. Six doses administered over 3 weeks (on days 1, 3, 5, 7, 14, 21). |
| Criteria for assessment | Safety and tolerability: Physical examinations, vital signs, adverse event monitoring and ECGs. Blood sampling for clinical safety (haematology, biochemistry), coagulation (PT, APTT and TT), urinalysis and complement assessments (Bb) |
| | Pharmacokinetic: Blood sampling for plasma ATL1103 levels at various time points over 7 days (Stage A) and 35 days (Stage B) |
| | Pharmacodynamic: |
| | IGF-I: Serum samples collected at least weekly to day 35. Exploratory PD: Blood sampling for GH, and the following for Stage B only: IGF-BP3, ALS, IGF-II, GHBP and *in vitro* mitogenic and apoptotic parameters on Days 1, 7, 21 and 28 of the study |
| Subject withdrawals | Two subjects withdrew from the study after the fifth dose of ATL1103 due to 1) withdrawal of consent 2) subject lost to follow up. No subject withdrew or was withdrawn for safety reasons. |
| Outcomes | Primary objective outcomes |
| | • ATL1103 was considered safe and generally well tolerated at the doses used in the study |
| | • There were no serious adverse events reported |
| | • There were 24 treatment-emergent adverse events (TEAE) in Stage A (19 in the 16 ATL1103-treated subjects, 5 in the 8 placebo-treated subjects), all reported as mild or moderate. In the ATL1103-treated subjects most common adverse events reported were pain at injection site (6), headache (5), influenza-like illness (2). |
| | • There were 25 TEAEs in Stage B (18 in the 8 ATL1103-treated subjects; 7 in the 4 placebo-treated subjects). All were reported as mild. In ATL1103-treated subjects the most commonly reported adverse events were injection site reactions (13). |
| | • Notably, although influenza- like illness (inc muscle aching and fever) was seen in two subjects after single doses of 400mg ATL1103, this was not seen after repeated doses of 250mg. |
| | • Increased ALT levels were reported for one subject in Stage B at Day 11. ALT levels returned to the normal range by pre-dose day 21 and remained within normal range throughout the rest of the study period. |
| | • A summary of the pharmacokinetic parameters is shown in Table 1. Secondary & Exploratory objectives outcomes |
| | The effect of ATL1103 on serum IGF-I levels and on the exploratory PD markers were determined as change from baseline levels. |
| | • For IGF-I there was a clear trend for mean levels to be lower than baseline on days D14, 21, 28, 35 of the study with a statistically significant effect reached by day 21 (Table 2) |
| | • No treatment-related effects were apparent in growth hormone levels (data not shown) |
| | • The inhibitory effect of ATL1103 on other exploratory PD markers is shown in Table 3. Of particular note is the ATL1103-related inhibition of circulating GHBP. GHBP is produced by cleavage from the GHr receptor, so reduction of circulating GHBP levels suggests that GHr expression is reduced. This provides support for ATL1103 working via an antisense mechanism of action. |
| | • IGF-BP3 and ALS reductions are consistent with the effect of ATL1103 on IGF-I (Table 3). |

**Table 4: Summary of pharmacokinetic parameters (mean ± SD)**

| | n | **Cmax (ng/mL)** | **Tmax (hr)** | **AUClast (hr^{∗}ng/mL** |
|---|---|---|---|---|
| **Single Dose** | | | | |
| 25 mg | 4 | 466 ± 136 | 3.25 ± 0.5 | 3711 ± 822 |
| 75 mg | 4 | 3139 ± 1576 | 3.25 ± 0.96 | 21342 ± 4755 |
| 250 mg | 4 | 12383 ± 3000 | 2.5 ± 1.29 | 88942 ± 11295 |
| 400 mg | 4 | 14343 ± 2823 | 3.25 ± 0.96 | 151123 ± 29434 |

| **Multiple dose** (250 mg; Pharmacokinetic population) | | | | |
|---|---|---|---|---|
| Day 1 | 6 | 8318 ± 2623 | 3.84 ± 1.49 | 58400 ± 11220 |
| Day 21 | 6 | 8557 ± 1431 | 3.00 ± 0.63 | 91870 ± 16795 |

**Table 5: Serum IGF-I levels in ATL1103-treated subjects in Stage B**

| Pre-dose IGF-I: 36.57 ± 13.16 nmol/L:mean ± SD | | |
|---|---|---|
| Study day | Change from pre-dose Mean ± SD (% change from pre-dose) | Probability < (-t)^{a} |
| Day 3 | + 2.65 ± 9.47 (12%) | > 0.500 |
| Day 5 | + 0.32 ± 7.42 (6%) | > 0.500 |
| Day 7 | + 0.77 ± 7.51 (6%) | > 0.500 |
| Day 14 | - 1.43 ± 7.19 (-2%) | 0.323 |
| Day 21 | - 3.40 ± 3.59 (-7%) | 0.034 |
| Day 28 | - 3.43 ± 7.53 (-7%) | 0.157 |
| Day 35 | - 1.9 ± 5.08 (-1%) | 0.201 |

| | | |
|---|---|---|
| ^{a} 1-sided t-test on change from pre-dose at each time point; alternate hypothesis is suppression Per protocol population; (n=6 at each time point) | | |

**Table 6: Exploratory PD assessments of ATL1103 subjects in Stage B**

| | Pre-dose value/Mean change from pre-dose (% change from pre-dose) | | | |
|---|---|---|---|---|
| | GHBP (pmol/L) | IGF-BP3 (ng/ml) | IGF-II (ng/ml) | ALS (mU/ml) |
| Pre-dose | 1053.8 ± 664 | 2906.2 ± 320.8 | 722.3 ± 113.8 | 1710.2 ± 305.4 |
| Day 7 | -31.2 (0.%) | -310.5** (-10.%) | -101.8*** (-14%) | -115.3 (- 5%) |
| Day 21 | -169.5** (-16%) | -2.28.3* (-8%) | -57.5 (-8%) | -160.7 (-9%) |
| Day 28 | -233.3* (-19%) | -160.5 (-5%) | -29.2 (-5%) | -188.7* (-10%) |

| | | | | |
|---|---|---|---|---|
| *P < 0.050, **P < 0.010, *** p < 0.001; 1-sided t-test on change from pre-dose at each time point; alternate hypothesis is suppression Per protocol population; (n=6 at each time point) | | | | |

### Example 2: ATL1103 monotherapy and combination mouse study

### Background and study design

- Normal female mice (N=8 per group) were injected for 3 weeks with saline, ATL1103 (2 doses), Somavert (2 doses) and octreotide-acetate (1 dose), and assessed when used alone (monotherapy) and in combination with ATL1103.
- Serum IGF-I values were monitored every week for 3 weeks and the relative liver GHR RNA and IGF-I RNA levels were determined at 3 weeks of dosing.
- Somavert and octreotide are current medicinal treatments for acromegaly and the doses used in this mouse study have previously been shown to be active in prior published rodent studies.
- ATL1103, at the higher dose used in this study, has previously been shown in mice to be active at 3 weeks, reducing GHr RNA and serum IGF-I at 3 weeks.
- In normal mice the liver is the main source of IGF-I in the blood contributing to about 70% of the serum IGF-I.
- The GHr and IGF-I RNA data were statistically analysed using one-way ANOVA followed by Dunnetts test when comparing treated to saline control. The sIGF-I data were statistically analyzed using one-way ANOVA and pair wise comparisons.
- The objective of the study was to assess the effects of monotherapy versus a combination approach (ATL1103 combined with the other existing treatments) in mice as a guide to the potential clinical benefit of combination treatments.

The main outcomes of the study for the monotherapy and combination treatments arms are as follows:

### Monotherapy Treatment Outcomes:

- ATL1103 (25mg/kg, 2 times weekly) reduced liver GHr RNA (99.7%, p=0.001), IGF-I RNA (76.2% , p=0.001), and serum IGF-I (44.5%, p=0.003) at week 3 compared to the saline control, with similar sIGF-I reductions as at 1 (36.5%, p=0.0070)and week 2 (41.5%, p=0.004) compared to control saline.
- Lower doses of ATL1103 (12mg/kg, 3 times in the first week, and then 2 times weekly for two weeks) reduce liver GHr RNA (99%, p=0.001), IGF-I RNA (64.3%, p=0.001) at week 3.
- Octreotide (1.25mg/kg twice daily) reduced liver GHr RNA (82.9%, p=0.001), IGF-I RNA(47.7% , p=0.05), and serum IGF-I(25.2%, p=0.03) at week 3 compared to the saline control, with similar sIGF-I reductions as early as week 1 (25.1%, p=0.012).
- ATL1103 reduces liver GHr RNA and IGF-I RNA to levels lower than that achieved with octreotide at the doses employed in this study.
- Somavert (20mg/kg, every other day) reduced liver GHr RNA (79.9%, p=0.01) and IGF-I RNA (43.2%, p=0.05), at week 3 compared to the saline control. (**NB**: The sIGF-I assay assessed sIGF-I levels in Somavert treated samples as showing > 2 fold increase at 1 week at the higher Somavert dose. Somavert would be expected to decrease sIGF-I in this study which suggests that there was an issue with the assay and so other sIGF-I data on Somavert has not been reported here). Somavert at the lower dose (5mg/kg, every other day) increased liver GHr RNA (23%, p=0.05), with no effect on liver IGF-I RNA compared to the saline control.
- ATL1103 reduces liver GHr RNA and IGF-I RNA levels to lower than that achieved with the high dose of Somavert employed in this study.

### Combination Treatment Outcomes

- ATL1103 (25mg/kg, 2 times weekly) combinations with octreotide (1.25mg/kg twice daily) significantly reduced GHr RNA(99.5%, p=0.001), IGF-I RNA (76%, p=0.001), and sIGF-I (42.3%, p=0.005), compared to saline control at week 3.
- The ATL1103 combination with octreotide demonstrated a greater activity compared to octreotide monotherapy on each of these parameters. The combination did not demonstrate additional activity compared to ATL1103 monotherapy at week 3 in this mouse study.
- ATL1103 (25mg/kg, 2 times weekly) combinations with octreotide (1.25mg/kg twice daily) at week 1 showed significantly reduced sIGF-I (44%, p<0.002), compared to baseline control levels. ATL1103 (25mg/kg, 2 times weekly) monotherapy at week 1 showed reduced sIGF-I by 25%, p<0.013) compared to baseline control levels and octreotide (1.25mg/kg twice daily) reduced sIGF-I (15%, p<0.04), compared to baseline control levels.

- In the first week the ATL1103 combination with octreotide showed ATL1103 was significantly able to further reduce the sIGF-I reduction of octreotide and octreotide was able to further reduce the sIGF-I reduction of ATL1103 at the single doses tested. This showed the combination had an additional effect compared to the monotherapy.
- The ATL1103 low dose combination with high active dose Somavert reduced liver GHr RNA (99.5%, p=0.001), liver IGF-I RNA (86.6%, p=0.001), compared to saline at week 3. The ATL1103 high dose combination with Somavert reduced liver GHr RNA(99.8%, p=0.001), IGF-I RNA (87.6%, p=0.001), compared to control saline at week 3
- ATL1103 (lower and higher dose) combinations with the active higher dose Somavert (20mg/kg, every other day) at 3 weeks showed additional liver IGF-I RNA reductions that were statistically significant compared to either ATL1103 or Somavert monotherapy. This was observed using a factorial structure statistical analysis and a t-test. There was also a statistically significant additional reduction at the level of liver GHr RNA using the t-test.

The mouse ATL1103 combination studies with Somavert showed additional activity at the liver RNA level and support further explorations of the potential benefits of ATL1103.

### Example 3: Co-administration of an antisense oligonucleotide and Sandostatin-LAR including in subjects in need of serum IGF-I reduction.

ATL1103 is to be subcutaneously administered at 250 mg per day, six times over 3 weeks on days 1, 3, 5, 7, 14 and 21 or 250mg twice weekly for 21 days, and once or twice weekly at 250mg for a further 5 weeks. Sandostatin-LAR will be dosed at 20 mg every 28 days over the same 8 week period.

Control group: Sandostatin-LAR is to be administered alone for a similar 8 week period every 28 days to normal volunteers or subjects in need of reduction of serum IGF-I.

Pharmacodynamic and pharmacological effects will be assessed as described in Example 1 with similar assays and additional assays where useful, for example, for serum IGF-I and GH.

Treatment is to be continued with the same or increased or lower doses of Sandostatin-LAR, and with the same or increased or lower doses of ATL1103, and administered with the same or increased or lower dosing frequency to achieve the desired target serum IGF-I levels, and optionally, GH levels.

### Example4: Co-administration of an antisense oligonucleotide and Sandostatin-LAR for the treatment of acromegaly.

Groups of up to 15 acromegalics are to be maintained on their Sandostatin-LAR dose and additionally dosed with ATL1103 as described in Example 1.

Treatment is to be continued with the same or increased or lower doses of Sandostatin-LAR, and with the same or increased or lower doses of ATL1103, and administered with the same or increased or lower dosing frequency to achieve the desired target serum IGF-I levels and optionally, GH levels.

### Example 5: Co-administration of an antisense oligonucleotide and Lanreotide - LAR (Autogel) for the treatment of acromegaly

Groups of 15 acromegalics are to be dosed with 200 mg ATL1103 once or twice weekly dosing for 13 weeks in patients already on (i) 90 mg once every 28 days Lanreotide-LAR doses or (ii) 120 mg once every 28 day Lanreotide-LAR on the same days as ATL1103.

Treatment is to be continued with the same or increased or lower doses of Lanreotide-LAR doses, and with the same or increased or lower doses of ATL1103, and administered with the same or increased or lower dosing frequency to achieve the desired target serum IGF-I levels, and optionally GH levels.

### Example 6: Co-administration of an antisense oligonucleotide and Octreotide-LAR for the treatment of diabetic retinopathy

Groups of 15 patients with diabetic retinopathy are to be dosed with 200 mg ATL1103 once or twice weekly dosing for 13 weeks and either (i) 20 mg once every 28 days with Octreotide-LAR or (ii) 30 mg once every 28 days with Octreotide-LAR for 13 weeks on the same days as ATL1103.

Control groups: ATL1103 or Octreotide-LAR is to be administered alone for a similar 13 week period to diabetic retinopathy patients.

Treatment is to be continued with the same or increased or lower doses of Octreotide-LAR, and with the same or increased or lower doses of ATL1103, and administered with the same or increased or lower dosing frequency to achieve the desired target IGF-I levels, and optionally GH levels and outcomes in retinal disease.

### Example 7: Co-administration of an antisense oligonucleotide and Lanreotide-LAR for the treatment of cancer

Groups of 15 patients with cancer associated with increased IGF-I are to be dosed with 200 mg ATL1103 once or twice weekly dosing for 13 weeks and either (i) 90 mg once every 28 days with Lanreotide-LAR or (ii) 120 mg every 28 days with Lanreotide-LAR for 13 weeks

Control groups: ATL1103 or Lanreotide-LAR is to be administered alone, with patients standard medication, for a similar 13 week period to cancer patients.

Treatment is to be continued with the same or increased or lower doses of Lanreotide-LAR, and with the same or increased or lower doses of ATL1103, and administered with the same or increased or lower dosing frequency to achieve the desired target IGF-I levels, and optionally GH levels and outcomes in cancer.

### Example 8: Co-administration of an antisense oligonucleotide and Somatostatin analogue for the treatment of acromegaly

Somatostatin Analogue will be dosed at the doses acromegaly patients are currently using for their treatment, for example, 20, 30, mg/28days or more Octreotide-LAR, or 90, 120mg/28 days with Lanreotie-LAR, or 40 to 60mg Pasireotide-LAR.

ATL1103 is to be subcutaneously administered at, 250 mg per day, six times over 3 weeks on days 1, 3, 5, 7, 14 and 21 or 250mg twice weekly for 21 days, and once or twice weekly at 250mg for a further 5 weeks.

Pharmacodynamic and pharmacological effects will be assessed as described in Example 1 with similar assays and additional assays where useful, for example, for serum IGF-I and GH.

Treatment is to be continued with the same or increased or lower doses of Somatostatin analogue, and with the same or increased or lower doses of ATL1103, and administered with the same or increased or lower dosing frequency to achieve the desired target serum IGF-I and, optionally, GH levels.

### Example 9: Co-administration of an antisense oligonucleotide and Somatostatin analogue for the treatment of acromegaly

Somatostatin analogue will be dosed subcutaneously, or intramuscularly at the doses acromegaly patients are currently using for their treatment for example as outlined in the Example above.

ATL1103 is to be subcutaneously administered at 100, 150, 200, 250, 300, 350 or 400 mg over 3 weeks once or twice weekly or until a cumulative dose of ∼ 1200-1800 mg.

Pharmacodynamic and pharmacological effects will be assessed as described in Example 1 with similar assays and additional assays where useful, for example, for serum IGF-I and GH.

Treatment is to be continued with the same or increased or lower doses of Somatostatin analogue, and with the same or increased or lower doses of ATL1103, and administered with the same or increased or lower dosing frequency to achieve the desired target serum IGF-I and, optionally, GH levels.

### Example 10: Co-administration of an antisense oligonucleotide and Somatostatin analogue for the treatment of acromegaly

Somatostatin analogue will be dosed subcutaneously or intramuscularly at the doses acromegaly patients are currently using for their treatment, for example, as outlined in the Examples above.

ATL1103 is to be subcutaneously administered at 100, 150, 200, 250, 300, 350 or 400 mg over 4 weeks once or twice weekly, or over 6 weeks once or twice weekly, or over 8 weeks once or twice weekly, or over 12 weeks once or twice weekly.

Pharmacodynamic and pharmacological effects will be assessed as described in Example 1 with similar assays and additional assays where useful, for example, for serum IGF-I and GH.

Treatment is to be continued with the same or increased or lower doses of Somatostatin analogue, and with the same or increased or lower doses of ATL1103, and administered with the same or increased or lower dosing frequency to achieve the desired target serum IGF-I and, optionally, GH levels.

### Example 11: ATL1103 is effective at reducing IGF-1 levels in subjects with acromegaly

The Phase II trial of ATL1103 was a randomised, open-label, parallel group study of the safety, tolerability, pharmacokinetics and efficacy of two subcantaneous dosing regimens at ATL1103 in 24 adult patients with acromegaly dosed in ATL1103 for 13 weeks (3 months) with two months of follow-up. Two ATL1103 dosing regimens were tested (a) 200 mg 3 times in the first week then once weekly therafter (200 mg/week) or (b) 200 mg 3 times in the first week then twice weekly thereafter (400 mg/week). The primary endpoints or main purposes of the trial were (i) to evaluate the safety and tolerability of ATL1103 in patients with acromegaly, and (ii) to evaluate the single dose and multiple dose pharmacokinetic profiles of ATL1103 via the subcutaneous route in patients with acromegaly. A secondary, but important endpoint that is also on the trial protocol was the evaluation of ATL1103's effect on serum insulin like growth factor I (IGF-I) levels in patients. The secondary endpoint was the average percentage reduction in serum IGF-I levels at the end of treatment compared to baseline levels for each of the two dosing regimens used in the Phase II study.

In the 4 patients who received the 400 mg per week dose, ATL1103 reduced sIGF-I levels consistently and by an average (mean) of 30% (range 4% to 48%) at week 14 (one week past the last dose) which is the primary efficacy time point for the trial. sIGF-I levels were reduced by a mean of 38% (28-48%) in the 3 patients who had lower body weights (58-83 kg at screening) and thereby received a relatively higher dose per kg bodyweight of between approximately 4.8 and 6.9 mg/kg. The one patient showing the lowest sIGF-I reduction at week 14 had the highest body weight (132 kg at screening).

At the 200 mg per week dose, no consistent reduction in mean sIGF-I levels was observed at week 14, although some sIGF-I reduction was noted in individual patients.

The time course data (see Figure 1) at the twice weekly 200mg (400 mg per week) dose generally shows a progressive rate of reduction in sIGF-I over the dosing period. This is further support for the therapeutic action of ATL1103 observed in this trial and suggests that continued dosing of ATL1103 at the 400 mg per week dose for longer than 3 months could result in additional reductions in sIGF-I.

### REFERENCES

Altschul et al., J. Mol. Biol. (1990) 215:403-410
Brazeau et al., Science (1973) 179:77
Elbashir et al., Nature (2001a) 411:494-498
Elbashir et al., Genes Dev. (2001b) 15:188-200
Englisch et al., Angewandte Chemie, International Edition (1991) 30:613
Epstein et al., Proc. Natl. Acad. Sci. U.S.A. (1985) 82:3688-3692
Fire et al., Nature (1998) 391:806-811
Giustina et al., Pituitary (2011) 14:125-133
Guo and Kempheus, Cell (1995) 81:611-620
Martin et al., Helv. Chim. Acta (1995) 78:486-504
Montgomery et al., Proc. Natl. Acad. Sci. USA. (1998) 95:15502-15507
Nielsen et al., Science (1991) 254, 1497-1500
Tabara et al., Science (1998) 282:430-431
Tijsterman et al., Science (2002) 295:694-697
Timmons et al., Gene (2001) 263:102-112
Timmons and Fire, Nature (1998) 395:854
Tuschl et al., Genes Dev. (1999) 13:3191-3197
Zhang and Madden, Genome Res. (1997) 7:649-656

## Claims

1. Octreotide, Lanreotide, Pasireotide or Octreolin in combination with an antisense oligonucleotide 15 to 30 nucleobases in length targeted to a nucleic acid encoding human Growth Hormone Receptor (GHR) for use in treating acromegaly in a subject having serum levels of insulin-like growth factor I (IGF-I) above normal despite Somatostatin agonist therapy, wherein said oligonucleotide:
(i) comprises at least an 8 consecutive nucleobase portion of SEQ ID NO: 6;
(ii) consists of a deoxynucleotide region flanked on both of the 5' and 3' ends with one or more 2'-O-modified sugar moieties;
(iii) comprises at least one 2'-O-methoxyethyl sugar moiety, at least one phosphorothioate internucleoside linkage, and at least one 5-methylcytosine.

2. Octreotide, Lanreotide, Pasireotide or Octreolin and the oligonucleotide for use according to claim 1, wherein the oligonucleotide consists of the nucleobase sequence of SEQ ID NO:6.

3. Octreotide, Lanreotide, Pasireotide or Octreolin and the oligonucleotide for use according to any one of claims 1 to 2, wherein the oligonucleotide consists of 20 linked nucleosides, wherein the oligonucleotide consists of a nucleobase of SEQ ID NO:6; and wherein the oligonucleotide consists of a ten deoxynucleotide region flanked on both the 5' end and the 3' end of said ten deoxynucleotide region with five 2'-O-(2-methoxyethyl) nucleotides, and wherein each internucleoside linkage in the oligonucleotide is a phosphorothioate linkage, and wherein each cytosine in said oligonucleotide is a 5-methylcytosine.

## Patentansprüche

1. Ocreotid, Lanreotid, Pasireotid oder Octreotid in Kombination mit einem Antisense-Oligonukleotid, das eine Länge von 15 bis 30 Nukleinbasen aufweist, gerichtet auf eine Nukleinsäure, die den menschlichen Wachstumshormon-Rezeptor (GHR) codiert, zur Verwendung beim Behandeln von Akromegalie in einer Person, die trotz einer Somatostatinagonistentherapie Serumspiegel des insulinähnlichen Wachstumsfaktors I (IGF-I) über normal aufweist, wobei das Oligonukleotid:
(i) zumindest 8 aufeinanderfolgende Nukleinbasenabschnitte der SEQ ID NO: 6 umfasst;
(ii) aus einer Desoxynukleotid-Region besteht, die sowohl an dem 5'- als auch dem 3'-Ende mit einer oder mehreren 2'-O-modifizierten Zuckereinheiten flankiert ist;
(iii) zumindest eine 2'-O-Methoxyethyl-Zuckereinheit, zumindest eine Phosphorthioat-Internukleosidverknüpfung und zumindest ein 5-Methylcytosin umfasst.

2. Ocreotid, Lanreotid, Pasireotid oder Octreotid und Oligonukleotid zur Verwendung nach Anspruch 1, wobei das Oligonukleotid aus der Nukleinbasensequenz von SEQ ID NO: 6 besteht.

3. Ocreotid, Lanreotid, Pasireotid oder Octreotid und Oligonukleotid zur Verwendung nach einem der Ansprüche 1 bis 2, wobei das Oligonukleotid aus 20 verknüpften Nukleosiden besteht, wobei das Oligonukleotid aus einer Nukleinbase der SEQ ID NO: 6 besteht und wobei das Oligonukleotid aus zehn Desoxynukleotidregionen besteht, die sowohl an dem 5'-Ende als auch dem 3'-Ende der zehn Desoxynukleotidregionen mit fünf 2'-O-(2-Methoxyethyl-)Nukleotiden flankiert sind, und wobei jede Internukleosidverknüpfung in dem Oligonukleotid eine Phosphorthioatverknüpfung ist und wobei jedes Cytosin in dem Oligonukleotid ein 5-Methylcytosin ist.

## Revendications

1. Octréotide, lanréotide, pasiréotide ou octréoline en combinaison avec un oligonucléotide antisens de 15 à 30 nucléobases de longueur ciblé sur un acide nucléique codant pour le récepteur d'hormone de croissance humaine (GHR) à utiliser dans le traitement de l'acromégalie chez un sujet ayant des taux sériques de facteur de croissance de type insuline I (IGF-I) supérieurs à la normale malgré un traitement par agoniste de la somatostatine, où ledit oligonucléotide :
(i) comprend au moins une portion de 8 nucléobases consécutives de SEQ ID NO:6 ;
(ii) est constitué d'une région désoxynucléotidique flanquée sur les deux extrémités 5' et 3' avec un ou plusieurs groupements sucre modifiés 2'-O ;
(iii) comprend au moins un groupement sucre 2'-O-méthoxyéthyle, au moins une liaison internucléosidique phosphorothioate et au moins une 5-méthylcytosine.

2. Octréotide, lanréotide, pasiréotide ou octréoline et oligonucléotide à utiliser selon la revendication 1, où l'oligonucléotide est constitué de la séquence de nucléobases de SEQ ID NO:6.

3. Octréotide, lanréotide, pasiréotide ou octréoline et oligonucléotide à utiliser selon l'une quelconque des revendications 1 à 2, où l'oligonucléotide est constitué de 20 nucléosides liés, où l'oligonucléotide est constitué d'une nucléobase de SEQ ID NO:6 ; et dans lesquels l'oligonucléotide est constitué d'une région de dix désoxynucléotides flanquée à la fois sur l'extrémité 5' et l'extrémité 3' de ladite région de dix désoxynucléotides avec cinq nucléotides 2'-O-(2-méthoxyéthyle), et où chaque liaison internucléosidique dans l'oligonucléotide est une liaison phosphorothioate, et dans lesquels chaque cytosine dans ledit oligonucléotide est une 5-méthylcytosine.


### SEQUENCE LISTING

<110> Antisense Therapeutics Ltd
<120> Combination Therapy
<130> 513110
<150> US 61/594532
   <151> 2012-02-03
<160> 154
<170> PatentIn version 3.5
<210> 1
   <211> 586
   <212> DNA
   <213> Homo sapiens
<400> 1
   cgatatgttc ccaactattc cactgagtcg cctgttcgat aacgcgatgc tgcgtgcgca      60
   tcgtctgcac caactggctt tcgacactta ccaggagttc gaagaagcat acatcccgaa     120
   agaacagaaa tacagcttcc ttcagaaccc acagacctcg ttgtgtttct ctgaaagtat     180
   cccgacccct tctaaccgcg aagagaccca gcagaaatcg aaccttgaac tgcttcgtat     240
   ctcgctgctt ctcattcagt cgtggctgga gccagtacag ttcctgcgtt cggttttcgc     300
   aaactcactg gtttacggtg cgtctgacag taacgtttac gacctgctga aagaccttga     360
   agaagggatc cagaccctga tgggtcgcct ggaagatggt tcaccacgca ctggtcagat     420
   cttcaaacag acttactcca aattcgatac taactctcat aacgatgatg ctctgctgaa     480
   aaactacggc ctgctgtact gtttccgtaa agatatggat aaagttgaaa ctttcctgcg     540
   tatcgttcag tgtcgttctg ttgaagggtc gtgtggcttc taatag                    586
<210> 2
   <211> 192
   <212> PRT
   <213> Homo sapiens
<400> 2
   Met Phe Pro Thr Ile Pro Leu Ser Arg Leu Phe Asp Asn Ala Met Leu
   1               5                   10                  15
   Arg Ala His Arg Leu His Gln Leu Ala Phe Asp Thr Tyr Gln Glu Phe
               20                  25                  30
   Glu Glu Ala Tyr Ile Pro Lys Glu Gln Lys Tyr Ser Phe Leu Gln Asn
           35                  40                  45
   Pro Gln Thr Ser Leu Cys Phe Ser Glu Ser Ile Pro Thr Pro Ser Asn
       50                  55                  60
   Arg Glu Glu Thr Gln Gln Lys Ser Asn Leu Glu Leu Leu Arg Ile Ser
   65 70 75 80
   Leu Leu Leu Ile Gln Ser Trp Leu Glu Pro Val Gln Phe Leu Arg Ser
   85 90 95
   Val Phe Ala Asn Ser Leu Val Tyr Gly Ala Ser Asp Ser Asn Val Tyr
   100 105 110
   Asp Leu Leu Lys Asp Leu Glu Glu Gly Ile Gln Thr Leu Met Gly Arg
   115 120 125
   Leu Glu Asp Gly Ser Pro Arg Thr Gly Gln Ile Phe Lys Gln Thr Tyr
   130 135 140
   Ser Lys Phe Asp Thr Asn Ser His Asn Asp Asp Ala Leu Leu Lys Asn
   145 150 155 160
   Tyr Gly Leu Leu Tyr Cys Phe Arg Lys Asp Met Asp Lys Val Glu Thr
   165 170 175
   Phe Leu Arg Ile Val Gln Cys Arg Ser Val Glu Gly Ser Cys Gly Phe
   180 185 190
<210> 3
   <211> 191
   <212> PRT
   <213> Artificial Sequence
<220>
   <223> Somavert
<400> 3
   Phe Pro Thr Ile Pro Leu Ser Arg Leu Phe Asp Asn Ala Met Leu Arg
   1 5 10 15
   Ala Asp Arg Leu Asn Gln Leu Ala Phe Asp Thr Tyr Gln Glu Phe Glu
   20 25 30
   Glu Ala Tyr Ile Pro Lys Glu Gln Lys Tyr Ser Phe Leu Gln Asn Pro
   35 40 45
   Gln Thr Ser Leu Cys Phe Ser Glu Ser Ile Pro Thr Pro Ser Asn Arg
   50 55 60
   Glu Glu Thr Gln Gln Lys Ser Asn Leu Glu Leu Leu Arg Ile Ser Leu
   65 70 75 80
   Leu Leu Ile Gln Ser Trp Leu Glu Pro Val Gln Phe Leu Arg Ser Val
                   85                  90                  95
   Phe Ala Asn Ser Leu Val Tyr Gly Ala Ser Asp Ser Asn Val Tyr Asp
               100                 105                 110
   Leu Leu Lys Asp Leu Gln Gln Lys Ile Gln Thr Leu Met Gly Arg Leu
           115                 120                 125
   Glu Asp Gly Ser Pro Arg Thr Gly Gln Ile Phe Lys Gln Thr Tyr Ser
       130                 135                 140
   Lys Phe Asp Thr Asn Ser His Asn Asp Asp Ala Leu Leu Lys Asn Tyr
   145                 150                 155                 160
   Gly Leu Leu Tyr Cys Phe Asn Ala Asp Met Ser Arg Val Ser Thr Phe
                   165                 170                 175
   Leu Arg Thr Val Gln Cys Arg Ser Val Glu Gly Ser Cys Gly Phe
               180                 185                 190
<210> 4
   <211> 4563
   <212> DNA
   <213> Homo sapiens
<400> 4
   cggcggcggc ggcggcagcg gcagcagcag ctgctacagt ggcggtggcg gcggcggctg      60
   ctgctgagcc cgggcggcgg cggggacccc gggctggggc cacgcgggcc ggaggccccg     120
   gcaccattgg ccccagcgca gacgcgaacc cgcgctctct gatcagaggc gaagctcgga     180
   ggtcctacag gtatggatct ctggcagctg ctgttgacct tggcactggc aggatcaagt     240
   gatgcttttt ctggaagtga ggccacagca gctatcctta gcagagcacc ctggagtctg     300
   caaagtgtta atccaggcct aaagacaaat tcttctaagg agcctaaatt caccaagtgc     360
   cgttcacctg agcgagagac tttttcatgc cactggacag atgaggttca tcatggtaca     420
   aagaacctag gacccataca gctgttctat accagaagga acactcaaga atggactcaa     480
   gaatggaaag aatgccctga ttatgtttct gctggggaaa acagctgtta ctttaattca     540
   tcgtttacct ccatctggat accttattgt atcaagctaa ctagcaatgg tggtacagtg     600
   gatgaaaagt gtttctctgt tgatgaaata gtgcaaccag atccacccat tgccctcaac     660
   tggactttac tgaacgtcag tttaactggg attcatgcag atatccaagt gagatgggaa     720
   gcaccacgca atgcagatat tcagaaagga tggatggttc tggagtatga acttcaatac     780
   aaagaagtaa atgaaactaa atggaaaatg atggacccta tattgacaac atcagttcca     840
   gtgtactcat tgaaagtgga taaggaatat gaagtgcgtg tgagatccaa acaacgaaac 900
   tctggaaatt atggcgagtt cagtgaggtg ctctatgtaa cacttcctca gatgagccaa 960
   tttacatgtg aagaagattt ctactttcca tggctcttaa ttattatctt tggaatattt 1020
   gggctaacag tgatgctatt tgtattctta ttttctaaac agcaaaggat taaaatgctg 1080
   attctgcccc cagttccagt tccaaagatt aaaggaatcg atccagatct cctcaaggaa 1140
   ggaaaattag aggaggtgaa cacaatctta gccattcatg atagctataa acccgaattc 1200
   cacagtgatg actcttgggt tgaatttatt gagctagata ttgatgagcc agatgaaaag 1260
   actgaggaat cagacacaga cagacttcta agcagtgacc atgagaaatc acatagtaac 1320
   ctaggggtga aggatggcga ctctggacgt accagctgtt gtgaacctga cattctggag 1380
   actgatttca atgccaatga catacatgag ggtacctcag aggttgctca gccacagagg 1440
   ttaaaagggg aagcagatct cttatgcctt gaccagaaga atcaaaataa ctcaccttat 1500
   catgatgctt gccctgctac tcagcagccc agtgttatcc aagcagagaa aaacaaacca 1560
   caaccacttc ctactgaagg agctgagtca actcaccaag ctgcccatat tcagctaagc 1620
   aatccaagtt cactgtcaaa catcgacttt tatgcccagg tgagcgacat tacaccagca 1680
   ggtagtgtgg tcctttcccc gggccaaaag aataaggcag ggatgtccca atgtgacatg 1740
   cacccggaaa tggtctcact ctgccaagaa aacttcctta tggacaatgc ctacttctgt 1800
   gaggcagatg ccaaaaagtg catccctgtg gctcctcaca tcaaggttga atcacacata 1860
   cagccaagct taaaccaaga ggacatttac atcaccacag aaagccttac cactgctgct 1920
   gggaggcctg ggacaggaga acatgttcca ggttctgaga tgcctgtccc agactatacc 1980
   tccattcata tagtacagtc cccacagggc ctcatactca atgcgactgc cttgcccttg 2040
   cctgacaaag agtttctctc atcatgtggc tatgtgagca cagaccaact gaacaaaatc 2100
   atgccttagc ctttctttgg tttcccaaga gctacgtatt taatagcaaa gaattgactg 2160
   gggcaataac gtttaagcca aaacaatgtt taaacctttt ttgggggagt gacaggatgg 2220
   ggtatggatt ctaaaatgcc ttttcccaaa atgttgaaat atgatgttaa aaaaataaga 2280
   agaatgctta atcagataga tattcctatt gtgcaatgta aatattttaa agaattgtgt 2340
   cagactgttt agtagcagtg attgtcttaa tattgtgggt gttaattttt gatactaagc 2400
   attgaatggc tatgttttta atgtatagta aatcacgctt tttgaaaaag cgaaaaaatc 2460
   aggtggcttt tgcggttcag gaaaattgaa tgcaaaccat agcacaggct aattttttgt 2520
   tgtttcttaa ataagaaact tttttattta aaaaactaaa aactagaggt gagaaattta 2580
   aactataagc aagaaggcaa aaatagtttg gatatgtaaa acatttattt tgacataaag 2640
   ttgataaaga ttttttaata atttagactt caagcatggc tattttatat tacactacac 2700
   actgtgtact gcagttggta tgacccctct aaggagtgta gcaactacag tctaaagctg 2760
   gtttaatgtt ttggccaatg cacctaaaga aaaacaaact cgttttttac aaagcccttt 2820
   tatacctccc cagactcctt caacaattct aaaatgattg tagtaatctg cattattgga 2880
   atataattgt tttatctgaa tttttaaaca agtatttgtt aatttagaaa actttaaagc 2940
   gtttgcacag atcaacttac caggcaccaa aagaagtaaa agcaaaaaag aaaacctttc 3000
   ttcaccaaat cttggttgat gccaaaaaaa aatacatgct aagagaagta gaaatcatag 3060
   ctggttcaca ctgaccaaga tacttaagtg ctgcaattgc acgcggagtg agttttttag 3120
   tgcgtgcaga tggtgagaga taagatctat agcctctgca gcggaatctg ttcacaccca 3180
   acttggtttt gctacataat tatccaggaa gggaataagg tacaagaagc attttgtaag 3240
   ttgaagcaaa tcgaatgaaa ttaactgggt aatgaaacaa agagttcaag aaataagttt 3300
   ttgtttcaca gcctataacc agacacatac tcatttttca tgataatgaa cagaacatag 3360
   acagaagaaa caaggttttc agtccccaca gataactgaa aattatttaa accgctaaaa 3420
   gaaactttct ttctcactaa atcttttata ggatttattt aaaatagcaa aagaagaagt 3480
   ttcatcattt tttacttcct ctctgagtgg actggcctca aagcaagcat tcagaagaaa 3540
   aagaagcaac ctcagtaatt tagaaatcat tttgcaatcc cttaatatcc taaacatcat 3600
   tcatttttgt tgttgttgtt gttgttgaga cagagtctcg ctctgtcgcc aggctagagt 3660
   gcggtggcgc gatcttgact cactgcaatc tccacctccc acaggttcag gcgattcccg 3720
   tgcctcagcc tcctgagtag ctgggactac aggcacgcac caccatgcca ggctaatttt 3780
   tttgtatttt agcagagacg gggtttcacc atgttggcca ggatggtctc gatctcctga 3840
   cctcgtgatc cacccgactc ggcctcccaa agtgctggga ttacaggtgt aagccaccgt 3900
   gcccagccct aaacatcatt cttgagagca ttgggatatc tcctgaaaag gtttatgaaa 3960
   aagaagaatc tcatctcagt gaagaatact tctcattttt taaaaaagct taaaactttg 4020
   aagttagctt taacttaaat agtatttccc atttatcgca gacctttttt aggaagcaag 4080
   cttaatggct gataatttta aattctctct cttgcaggaa ggactatgaa aagctagaat 4140
   tgagtgttta aagttcaaca tgttatttgt aatagatgtt tgatagattt tctgctactt 4200
   tgctgctatg gttttctcca agagctacat aatttagttt catataaagt atcatcagtg 4260
   tagaacctaa ttcaattcaa agctgtgtgt ttggaagact atcttactat ttcacaacag 4320
   cctgacaaca tttctatagc caaaaatagc taaatacctc aatcagtctc agaatgtcat 4380
   tttggtactt tggtggccac ataagccatt attcactagt atgactagtt gtgtctggca 4440
   gtttatattt aactctcttt atgtctgtgg attttttcct tcaaagttta ataaatttat 4500
   tttcttggat tcctgatagt gtgcttctgt tatcaaacac caacataaaa atgatctaaa 4560
   cca 4563
<210> 5
   <211> 304901
   <212> DNA
   <213> Homo sapiens
<400> 5
<210> 6
   <211> 20
   <212> DNA
   <213> Artificial Sequence
<220>
   <223> oligonucleotide
<400> 6
   tcagggcatt ctttccattc 20
<210> 7
   <211> 20
   <212> DNA
   <213> Artificial Sequence
<220>
   <223> oligonucleotide
<400> 7
   cataatcagg gcattctttc 20
<210> 8
   <211> 20
   <212> **DNA**
   <213> Artificial Sequence
<220>
   <223> oligonucleotide
<400> 8
   cctttaatct ttggaactgg 20
<210> 9
   <211> 20
   <212> DNA
   <213> Artificial Sequence
<220>
   <223> oligonucleotide
<400> 9
   tcatcaatat ctagctcaat 20
<210> 10
   <211> 20
   <212> DNA
   <213> Artificial Sequence
<220>
   <223> oligonucleotide
<400> 10
   cttagaagtc tgtctgtgtc 20
<210> 11
   <211> 20
   <212> DNA
   <213> Artificial Sequence
<220>
   <223> oligonucleotide
<400> 11
   cctgctggtg taatgtcgct 20
<210> 12
   <211> 20
   <212> DNA
   <213> Artificial Sequence
<220>
   <223> oligonucleotide
<400> 12
   atgtaaatgt cctcttggtt 20
<210> 13
   <211> 20
   <212> DNA
   <213> Artificial Sequence
<220>
   <223> oligonucleotide
<400> 13
   tggtgatgta aatgtcctct 20
<210> 14
   <211> 20
   <212> DNA
   <213> Artificial Sequence
<220>
   <223> oligonucleotide
<400> 14
   ttctgtggtg atgtaaatgt 20
<210> 15
   <211> 20
   <212> DNA
   <213> Artificial Sequence
<220>
   <223> oligonucleotide
<400> 15
   aggctttctg tggtgatgta 20
<210> 16
   <211> 20
   <212> DNA
   <213> Artificial Sequence
<220>
   <223> oligonucleotide
<400> 16
   tggtaaggct ttctgtggtg 20
<210> 17
   <211> 20
   <212> DNA
   <213> Artificial Sequence
<220>
   <223> oligonucleotide
<400> 17
   agttggtctg tgctcacata 20
<210> 18
   <211> 20
   <212> DNA
   <213> Artificial Sequence
<220>
   <223> oligonucleotide
<400> 18
   tgttcagttg gtctgtgctc 20
<210> 19
   <211> 20
   <212> DNA
   <213> Artificial Sequence
<220>
   <223> oliognucleotide
<400> 19
   gcatgatttt gttcagttgg 20
<210> 20
   <211> 20
   <212> **DNA**
   <213> Artificial Sequence
<220>
   <223> oligonucleotide
<400> 20
   tataaaaggg ctttgtaaaa 20
<210> 21
   <211> 20
   <212> **DNA**
   <213> Artificial Sequence
<220>
   <223> oligonucleotide
<400> 21
   catagcagca aagtagcaga 20
<210> 22
   <211> 20
   <212> **DNA**
   <213> Artificial Sequence
<220>
   <223> oligonucleotide
<400> 22
   gctatttttg gctatagaaa 20
<210> 23
   <211> 20
   <212> **DNA**
   <213> Artificial Sequence
<220>
   <223> oligonucleotide
<400> 23
   gattgaggta tttagctatt 20
<210> 24
   <211> 20
   <212> **DNA**
   <213> Artificial Sequence
<220>
   <223> oligonucleotide
<400> 24
   gatccatacc tgtaggacct 20
<210> 25
   <211> 20
   <212> **DNA**
   <213> Artificial Sequence
<220>
   <223> oligonucleotide
<400> 25
   ccagagatcc atacctgtag 20
<210> 26
   <211> 20
   <212> **DNA**
   <213> Artificial Sequence
<220>
   <223> oligonucleotide
<400> 26
   tgctaaggat agctgctgtg 20
<210> 27
   <211> 20
   <212> **DNA**
   <213> Artificial Sequence
<220>
   <223> oligonucleotide
<400> 27
   ttgtctttag gcctggatta 20
<210> 28
   <211> 20
   <212> **DNA**
   <213> Artificial Sequence
<220>
   <223> oligonucleotide
<400> 28
   ttagaagaat ttgtctttag 20
<210> 29
   <211> 20
   <212> **DNA**
   <213> Artificial Sequence
<220>
   <223> oligonucleotide
<400> 29
   gtgaatttag gctccttaga 20
<210> 30
   <211> 20
   <212> DNA
   <213> Artificial Sequence
<220>
   <223> oligonucleotide
<400> 30
   gctgtatggg tcctaggttc 20
<210> 31
   <211> 20
   <212> DNA
   <213> Artificial Sequence
<220>
   <223> oligonucleotide
<400> 31
   taacagctgt tttccccagc 20
<210> 32
   <211> 20
   <212> DNA
   <213> Artificial Sequence
<220>
   <223> oligonucleotide
<400> 32
   tttcatccac tgtaccacca 20
<210> 33
   <211> 20
   <212> DNA
   <213> Artificial Sequence
<220>
   <223> oligonucleotide
<400> 33
   ttgcactatt tcatcaacag 20
<210> 34
   <211> 20
   <212> DNA
   <213> Artificial Sequence
<220>
   <223> oligonucleotide
<400> 34
   gggtggatct ggttgcacta 20
<210> 35
   <211> 20
   <212> DNA
   <213> Artificial Sequence
<220>
   <223> oligonucleotide
<400> 35
   attgcgtggt gcttcccatc 20
<210> 36
   <211> 20
   <212> DNA
   <213> Artificial Sequence
<220>
   <223> oligonucleotide
<400> 36
   tagggtccat cattttccat 20
<210> 37
   <211> 20
   <212> DNA
   <213> Artificial Sequence
<220>
   <223> oligonucleotide
<400> 37
   caatgagtac actggaactg 20
<210> 38
   <211> 20
   <212> DNA
   <213> Artificial Sequence
<220>
   <223> oligonucleotide
<400> 38
   aactcgccat aatttccaga 20
<210> 39
   <211> 20
   <212> DNA
   <213> Artificial Sequence
<220>
   <223> oligonucleotide
<400> 39
   agcccaaata ttccaaagat 20
<210> 40
   <211> 20
   <212> DNA
   <213> Artificial Sequence
<220>
   <223> oligonucleotide
<400> 40
   tcagcatttt aatcctttgc 20
<210> 41
   <211> 20
   <212> DNA
   <213> Artificial Sequence
<220>
   <223> oligonucleotide
<400> 41
   attttccttc cttgaggaga 20
<210> 42
   <211> 20
   <212> DNA
   <213> Artificial Sequence
<220>
   <223> oligonucleotide
<400> 42
   agattgtgtt cacctcctct 20
<210> 43
   <211> 20
   <212> DNA
   <213> Artificial Sequence
<220>
   <223> oligonucleotide
<400> 43
   aacccaagag tcatcactgt 20
<210> 44
   <211> 20
   <212> DNA
   <213> Artificial Sequence
<220>
   <223> oligonucleotide
<400> 44
   ctggctcatc aatatctagc 20
<210> 45
   <211> 20
   <212> DNA
   <213> Artificial Sequence
<220>
   <223> oligonucleotide
<400> 45
   tgtgtctgat tcctcagtct 20
<210> 46
   <211> 20
   <212> DNA
   <213> Artificial Sequence
<220>
   <223> oligonucleotide
<400> 46
   tatgtcattg gcattgaaat 20
<210> 47
   <211> 20
   <212> DNA
   <213> Artificial Sequence
<220>
   <223> oligonucleotide
<400> 47
   aaggcataag agatctgctt 20
<210> 48
   <211> 20
   <212> DNA
   <213> Artificial Sequence
<220>
   <223> oligonucleotide
<400> 48
   actcagctcc ttcagtagga 20
<210> 49
   <211> 20
   <212> DNA
   <213> Artificial Sequence
<220>
   <223> oligonucleotide
<400> 49
   ggacatccct gccttattct 20
<210> 50
   <211> 20
   <212> DNA
   <213> Artificial Sequence
<220>
   <223> oligonucleotide
<400> 50
   ggcattgtcc ataaggaagt 20
<210> 51
   <211> 20
   <212> DNA
   <213> Artificial Sequence
<220>
   <223> oligonucleotide
<400> 51
   actttttggc atctgcctca 20
<210> 52
   <211> 20
   <212> DNA
   <213> Artificial Sequence
<220>
   <223> oligonucleotide
<400> 52
   gatgcacttt ttggcatctg 20
<210> 53
   <211> 20
   <212> DNA
   <213> Artificial Sequence
<220>
   <223> oligonucleotide
<400> 53
   cagtcgcatt gagtatgagg 20
<210> 54
   <211> 20
   <212> DNA
   <213> Artificial Sequence
<220>
   <223> oligonucleotide
<400> 54
   ctctttgtca ggcaagggca 20
<210> 55
   <211> 20
   <212> DNA
   <213> Artificial Sequence
<220>
   <223> oligonucleotide
<400> 55
   gtgctcacat agccacatga 20
<210> 56
   <211> 20
   <212> DNA
   <213> Artificial Sequence
<220>
   <223> oligonucleotide
<400> 56
   aagaaaggct aaggcatgat 20
<210> 57
   <211> 20
   <212> DNA
   <213> Artificial Sequence
<220>
   <223> oligonucleotide
<400> 57
   aaatacgtag ctcttgggaa 20
<210> 58
   <211> 20
   <212> DNA
   <213> Artificial Sequence
<220>
   <223> oligonucleotide
<400> 58
   caatcactgc tactaaacag 20
<210> 59
   <211> 20
   <212> DNA
   <213> Artificial Sequence
<220>
   <223> oligonucleotide
<400> 59
   aaacatagcc attcaatgct 20
<210> 60
   <211> 20
   <212> DNA
   <213> Artificial Sequence
<220>
   <223> oligonucleotide
<400> 60
   gtgctatggt attgcattcaa 20
<210> 61
   <211> 20
   <212> DNA
   <213> Artificial Sequence
<220>
   <223> oligonucleotide
<400> 61
   gttttacata tccaaactat 20
<210> 62
   <211> 20
   <212> DNA
   <213> Artificial Sequence
<220>
   <223> oligonucleotide
<400> 62
   catcaaccaa gatttggtga 20
<210> 63
   <211> 20
   <212> DNA
   <213> Artificial Sequence
<220>
   <223> oligonucleotide
<400> 63
   gaggctatag atcttatctc 20
<210> 64
   <211> 20
   <212> DNA
   <213> Artificial Sequence
<220>
   <223> oligonucleotide
<400> 64
   tagtgagaaa gaaagtttct 20
<210> 65
   <211> 20
   <212> DNA
   <213> Artificial Sequence
<220>
   <223> oligonucleotide
<400> 65
   aatgctctca agaatgatgt 20
<210> 66
   <211> 20
   <212> DNA
   <213> Artificial Sequence
<220>
   <223> oligonucleotide
<400> 66
   acactcaatt ctagcttttc 20
<210> 67
   <211> 20
   <212> DNA
   <213> Artificial Sequence
<220>
   <223> oligonucleotide
<400> 67
   catctattac aaataacatg 20
<210> 68
   <211> 20
   <212> DNA
   <213> Artificial Sequence
<220>
   <223> oligonucleotide
<400> 68
   ctcttggaga aaaccatagc 20
<210> 69
   <211> 20
   <212> DNA
   <213> Artificial Sequence
<220>
   <223> oligonucleotide
<400> 69
   tctacactga tgatacttta 20
<210> 70
   <211> 20
   <212> DNA
   <213> Artificial Sequence
<220>
   <223> oligonucleotide
<400> 70
   cacagctttg aattgaatta 20
<210> 71
   <211> 20
   <212> DNA
   <213> Artificial Sequence
<220>
   <223> oligonucleotide
<400> 71
   agtcttccaa acacacagct 20
<210> 72
   <211> 20
   <212> DNA
   <213> Artificial Sequence
<220>
   <223> oligonucleotide
<400> 72
   aggctgttgt gaaatagtaa 20
<210> 73
   <211> 20
   <212> DNA
   <213> Artificial Sequence
<220>
   <223> oligonucleotide
<400> 73
   atagaaatgt tgtcaggctg 20
<210> 74
   <211> 20
   <212> DNA
   <213> Artificial Sequence
<220>
   <223> oligonucleotide
<400> 74
   ccaaaatgac attctgagac 20
<210> 75
   <211> 20
   <212> DNA
   <213> Artificial Sequence
<220>
   <223> oligonucleotide
<400> 75
   ataatggctt atgtggccac 20
<210> 76
   <211> 20
   <212> DNA
   <213> Artificial Sequence
<220>
   <223> oligonucleotide
<400> 76
   agttatgtga ccctgattga 20
<210> 77
   <211> 20
   <212> DNA
   <213> Artificial Sequence
<220>
   <223> oligonucleotide
<400> 77
   ttgagtgttc ctaaaatgaa 20
<210> 78
   <211> 20
   <212> DNA
   <213> Artificial Sequence
<220>
   <223> oligonucleotide
<400> 78
   atggaggctg gaggttcaaa 20
<210> 79
   <211> 20
   <212> DNA
   <213> Artificial Sequence
<220>
   <223> oligonucleotide
<400> 79
   tagggtccat ctttcaagac 20
<210> 80
   <211> 20
   <212> DNA
   <213> Artificial Sequence
<220>
   <223> oligonucleotide
<400> 80
   tctccagata gaatctaaac 20
<210> 81
   <211> 20
   <212> DNA
   <213> Artificial Sequence
<220>
   <223> oligonucleotide
<400> 81
   tccaaatatt ctggtacttt 20
<210> 82
   <211> 20
   <212> DNA
   <213> Artificial Sequence
<220>
   <223> oligonucleotide
<400> 82
   tattagttac cttgaggaga 20
<210> 83
   <211> 20
   <212> DNA
   <213> Artificial Sequence
<220>
   <223> oligonucleotide
<400> 83
   attttccttc ctagaaaata 20
<210> 84
   <211> 20
   <212> DNA
   <213> Artificial Sequence
<220>
   <223> target sequence
<400> 84
   gaatggaaag aatgccctga 20
<210> 85
   <211> 20
   <212> DNA
   <213> Artificial Sequence
<220>
   <223> target sequence
<400> 85
   gaaagaatgc cctgattatg 20
<210> 86
   <211> 20
   <212> DNA
   <213> Artificial Sequence
<220>
   <223> target sequence
<400> 86
   ccagttccaa agattaaagg 20
<210> 87
   <211> 20
   <212> DNA
   <213> Artificial Sequence
<220>
   <223> target sequence
<400> 87
   attgagctag atattgatga 20
<210> 88
   <211> 20
   <212> DNA
   <213> Artificial Sequence
<220>
   <223> target sequence
<400> 88
   gacacagaca gacttctaag 20
<210> 89
   <211> 20
   <212> DNA
   <213> Artificial Sequence
<220>
   <223> target sequence
<400> 89
   agcgacatta caccagcagg 20
<210> 90
   <211> 20
   <212> DNA
   <213> Artificial Sequence
<220>
   <223> target sequence
<400> 90
   aaccaagagg acatttacat 20
<210> 91
   <211> 20
   <212> DNA
   <213> Artificial Sequence
<220>
   <223> target sequence
<400> 91
   agaggacatt tacatcacca 20
<210> 92
   <211> 20
   <212> DNA
   <213> Artificial Sequence
<220>
   <223> target sequence
<400> 92
   acatttacat caccacagaa 20
<210> 93
   <211> 20
   <212> DNA
   <213> Artificial Sequence
<220>
   <223> target sequence
<400> 93
   tacatcacca cagaaagcct 20
<210> 94
   <211> 20
   <212> DNA
   <213> Artificial Sequence
<220>
   <223> target sequence
<400> 94
   caccacagaa agccttacca 20
<210> 95
   <211> 20
   <212> DNA
   <213> Artificial Sequence
<220>
   <223> target sequence
<400> 95
   tatgtgagca cagaccaact 20
<210> 96
   <211> 20
   <212> DNA
   <213> Artificial Sequence
<220>
   <223> target sequence
<400> 96
   gagcacagac caactgaaca 20
<210> 97
   <211> 20
   <212> DNA
   <213> Artificial Sequence
<220>
   <223> target sequence
<400> 97
   ccaactgaac aaaatcatgc 20
<210> 98
   <211> 20
   <212> DNA
   <213> Artificial Sequence
<220>
   <223> target sequence
<400> 98
   tctgctactt tgctgctatg 20
<210> 99
   <211> 20
   <212> DNA
   <213> Artificial Sequence
<220>
   <223> target sequence
<400> 99
   tttctatagc caaaaatagc 20
<210> 100
   <211> 20
   <212> DNA
   <213> Artificial Sequence
<220>
   <223> target sequence
<400> 100
   aatagctaaa tacctcaatc 20
<210> 101
   <211> 20
   <212> DNA
   <213> Artificial Sequence
<220>
   <223> target sequence
<400> 101
   aggtcctaca ggtatggatc 20
<210> 102
   <211> 20
   <212> DNA
   <213> Artificial Sequence
<220>
   <223> target sequence
<400> 102
   ctacaggtat ggatctctgg 20
<210> 103
   <211> 20
   <212> DNA
   <213> Artificial Sequence
<220>
   <223> target sequence
<400> 103
   cacagcagct atccttagca 20
<210> 104
   <211> 20
   <212> DNA
   <213> Artificial Sequence
<220>
   <223> target sequence
<400> 104
   taatccaggc ctaaagacaa 20
<210> 105
   <211> 20
   <212> DNA
   <213> Artificial Sequence
<220>
   <223> target sequence
<400> 105
   tctaaggagc ctaaattcac 20
<210> 106
   <211> 20
   <212> DNA
   <213> Artificial Sequence
<220>
   <223> target sequence
<400> 106
   gaacctagga cccatacagc 20
<210> 107
   <211> 20
   <212> DNA
   <213> Artificial Sequence
<220>
   <223> target sequence
<400> 107
   gctggggaaa acagctgtta 20
<210> 108
   <211> 20
   <212> DNA
   <213> Artificial Sequence
<220>
   <223> target sequence
<400> 108
   tggtggtaca gtggatgaaa 20
<210> 109
   <211> 20
   <212> DNA
   <213> Artificial Sequence
<220>
   <223> target sequence
<400> 109
   ctgttgatga aatagtgcaa 20
<210> 110
   <211> 20
   <212> DNA
   <213> Artificial Sequence
<220>
   <223> target sequence
<400> 110
   tagtgcaacc agatccaccc 20
<210> 111
   <211> 20
   <212> DNA
   <213> Artificial Sequence
<220>
   <223> target sequence
<400> 111
   gatgggaagc accacgcaat 20
<210> 112
   <211> 20
   <212> DNA
   <213> Artificial Sequence
<220>
   <223> target sequence
<400> 112
   atggaaaatg atggacccta 20
<210> 113
   <211> 20
   <212> DNA
   <213> Artificial Sequence
<220>
   <223> target sequence
<400> 113
   cagttccagt gtactcattg 20
<210> 114
   <211> 20
   <212> DNA
   <213> Artificial Sequence
<220>
   <223> target sequence
<400> 114
   tctggaaatt atggcgagtt 20
<210> 115
   <211> 20
   <212> DNA
   <213> Artificial Sequence
<220>
   <223> target sequence
<400> 115
   atctttggaa tatttgggct 20
<210> 116
   <211> 20
   <212> DNA
   <213> Artificial Sequence
<220>
   <223> target sequence
<400> 116
   gcaaaggatt aaaatgctga 20
<210> 117
   <211> 20
   <212> DNA
   <213> Artificial Sequence
<220>
   <223> target sequence
<400> 117
   tctcctcaag gaaggaaaat 20
<210> 118
   <211> 20
   <212> DNA
   <213> Artificial Sequence
<220>
   <223> target sequence
<400> 118
   agaggaggtg aacacaatct 20
<210> 119
   <211> 20
   <212> DNA
   <213> Artificial Sequence
<220>
   <223> target sequence
<400> 119
   acagtgatga ctcttgggtt 20
<210> 120
   <211> 20
   <212> DNA
   <213> Artificial Sequence
<220>
   <223> target sequence
<400> 120
   gctagatatt gatgagccag 20
<210> 121
<400> 121
   000
<210> 122
   <211> 20
   <212> DNA
   <213> Artificial Sequence
<220>
   <223> target sequence
<400> 122
   atttcaatgc caatgacata 20
<210> 123
   <211> 20
   <212> DNA
   <213> Artificial Sequence
<220>
   <223> target sequence
<400> 123
   aagcagatct cttatgcctt 20
<210> 124
   <211> 20
   <212> DNA
   <213> Artificial Sequence
<220>
   <223> target sequence
<400> 124
   tcctactgaa ggagctgagt 20
<210> 125
   <211> 20
   <212> DNA
   <213> Artificial Sequence
<220>
   <223> target sequence
<400> 125
   agaataaggc agggatgtcc 20
<210> 126
   <211> 20
   <212> DNA
   <213> Artificial Sequence
<220>
   <223> target sequence
<400> 126
   acttccttat ggacaatgcc 20
<210> 127
   <211> 20
   <212> DNA
   <213> Artificial Sequence
<220>
   <223> target sequence
<400> 127
   tgaggcagat gccaaaaagt 20
<210> 128
   <211> 20
   <212> DNA
   <213> Artificial Sequence
<220>
   <223> target sequence
<400> 128
   cagatgccaa aaagtgcatc 20
<210> 129
   <211> 20
   <212> DNA
   <213> Artificial Sequence
<220>
   <223> target sequence
<400> 129
   cctcatactc aatgcgactg 20
<210> 130
   <211> 20
   <212> DNA
   <213> Artificial Sequence
<220>
   <223> target sequence
<400> 130
   tgcccttgcc tgacaaagag 20
<210> 131
   <211> 20
   <212> DNA
   <213> Artificial Sequence
<220>
   <223> target sequence
<400> 131
   tcatgtggct atgtgagcac 20
<210> 132
<400> 132
   000
<210> 133
   <211> 20
   <212> DNA
   <213> Artificial Sequence
<220>
   <223> target sequence
<400> 133
   ttcccaagag ctacgtattt 20
<210> 134
   <211> 20
   <212> DNA
   <213> Artificial Sequence
<220>
   <223> target sequence
<400> 134
   ctgtttagta gcagtgattg 20
<210> 135
   <211> 20
   <212> DNA
   <213> Artificial Sequence
<220>
   <223> target sequence
<400> 135
   ttgaatgcaa accatagcac 20
<210> 136
   <211> 20
   <212> DNA
   <213> Artificial Sequence
<220>
   <223> target sequence
<400> 136
   atagtttgga tatgtaaaac 20
<210> 137
   <211> 20
   <212> DNA
   <213> Artificial Sequence
<220>
   <223> target sequence
<400> 137
   tcaccaaatc ttggttgatg 20
<210> 138
   <211> 20
   <212> DNA
   <213> Artificial Sequence
<220>
   <223> target sequence
<400> 138
   gagataagat ctatagcctc 20
<210> 139
   <211> 20
   <212> DNA
   <213> Artificial Sequence
<220>
   <223> target sequence
<400> 139
   agaaactttc tttctcacta 20
<210> 140
   <211> 20
   <212> DNA
   <213> Artificial Sequence
<220>
   <223> target sequence
<400> 140
   acatcattct tgagagcatt 20
<210> 141
   <211> 20
   <212> DNA
   <213> Artificial Sequence
<220>
   <223> target sequence
<400> 141
   gaaaagctag aattgagtgt 20
<210> 142
   <211> 20
   <212> DNA
   <213> Artificial Sequence
<220>
   <223> target sequence
<400> 142
   gctatggttt tctccaagag 20
<210> 143
   <211> 20
   <212> DNA
   <213> Artificial Sequence
<220>
   <223> target sequence
<400> 143
   taaagtatca tcagtgtaga 20
<210> 144
   <211> 20
   <212> DNA
   <213> Artificial Sequence
<220>
   <223> target sequence
<400> 144
   taattcaatt caaagctgtg 20
<210> 145
   <211> 20
   <212> DNA
   <213> Artificial Sequence
<220>
   <223> target sequence
<400> 145
   agctgtgtgt ttggaagact 20
<210> 146
   <211> 20
   <212> DNA
   <213> Artificial Sequence
<220>
   <223> target sequence
<400> 146
   ttactatttc acaacagcct 20
<210> 147
   <211> 20
   <212> DNA
   <213> Artificial Sequence
<220>
   <223> target sequence
<400> 147
   cagcctgaca acatttctat 20
<210> 148
   <211> 20
   <212> DNA
   <213> Artificial Sequence
<220>
   <223> target sequence
<400> 148
   gtctcagaat gtcattttgg 20
<210> 149
   <211> 20
   <212> DNA
   <213> Artificial Sequence
<220>
   <223> target sequence
<400> 149
   gtggccacat aagccattat 20
<210> 150
   <211> 20
   <212> DNA
   <213> Artificial Sequence
<220>
   <223> target sequence
<400> 150
   tcaatcaggg tcacataact 20
<210> 151
   <211> 20
   <212> DNA
   <213> Artificial Sequence
<220>
   <223> target sequence
<400> 151
   tttgaacctc cagcctccat 20
<210> 152
   <211> 20
   <212> DNA
   <213> Artificial Sequence
<220>
   <223> target sequence
<400> 152
   gtcttgaaag atggacccta 20
<210> 153
   <211> 20
   <212> DNA
   <213> Artificial Sequence
<220>
   <223> target sequence
<400> 153
   gtttagattc tatctggaga 20
<210> 154
   <211> 20
   <212> DNA
   <213> Artificial Sequence
<220>
   <223> target sequence
<400> 154
   aaagtaccag aatatttgga 20